# EUROPEAN PATENT APPLICATION

(11) **EP 0 714 896 A1**
(43) Date of publication of application: **05.06.1996**
(21) Application number: 95921844.7
(22) Date of filing: 13.06.1995
(51) Int. Cl.: C07D 403/12, C07D 403/14, A61K 31/505, C07D 401/14

(54) **INDOL DERIVATIVES USEFUL FOR THE TREATMENT OF MIGRAINE, COMPOSITION AND UTILIZATION**

(30) Priority: 21.06.1994 ES 9400134
(71) Applicant: VITA-INVEST, S.A., E-08970 Sant Joan Despi (Barcelona) (ES)
(72) Inventor: DEL CASTILLO NIETO, Juan Carlos, E-08027 Barcelona (ES); CALDERO GES, José Maria, E-08034 Barcelona (ES); ROCA ACIN, Juan, E-08006 Barcelona (ES); GIMENEZ GUASCH, Fernando, E-08028 Barcelona (ES); BOSCH ROVIRA, Anna, E-08017 Barcelona (ES); DALMASES BARJOAN, Pedro, E-08980 Sant Feliu de Llobregat (ES); HUGUET CLOTET, Juan, E-08970 Sant Joan Despi (ES); MARQUILLAS OLONDRIZ, Francisco, E-08034 Barcelona (ES)
(74) Representative: Curell Sunol, Jorge
(86) International application number: ES9500074
(87) International publication number: WO9535293

(57) **Abstract**

Indole derivatives having the Formula (I) wherein R₁ is H, alkyl or acyl group; R₂ is H, halogen or alkyl, hydroxy, alkoxy; ciano, carboxamide, carboxyl, alkoxicarbonyl, phenyl, phenyloxy or a group -(CH₂)ₙ-R₆, (n=1-3), and R₆ is a group ciano, nitro, phenyl, carboxyl, -CO₂R₇; -CONR₇R₈; -SO₂NR₇R₈; -COR₇; -SO₂R₇, R₇ and R₈ being H or alkyl; R₇ and R₈, together with N, can form a cycle of 4-7 links; R₃, R₄ and R₅ are H, halogen or a group alkyl, phenyl, substituted phenyl, hydroxy, alcoxy, phenyloxy or benziloxy; NR₁₁R₁₂ is H or a group ciano, nitro, carboxyl, alcoxycarbonyl, carboxamido or a group R₃; A is an alkyliden group -(CH₂)-ₘ, (m = 1-5), or alkenyl group; B is a piperazinoring (a), aminopyrrolidinoring (b); pyrrolidinamino ring (c); piperidinoring (d) or ethylendiamino -NR₉-CH₂-CH₂-NR₁₀-, R₉ and R₁₀ being H or an alkyl group. The compounds are useful for the treatment of migraine.

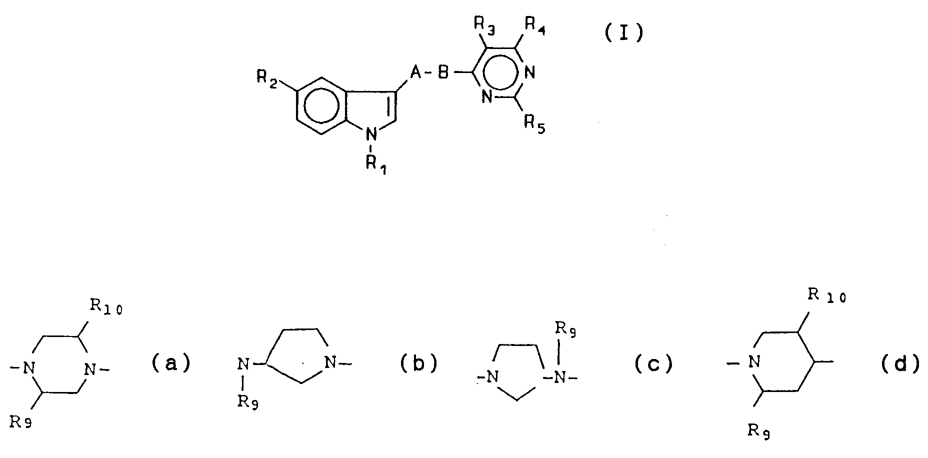

## Description

### Field of the Invention

The invention relates to indole derivatives useful for the treatment of migraine, of general Formula I 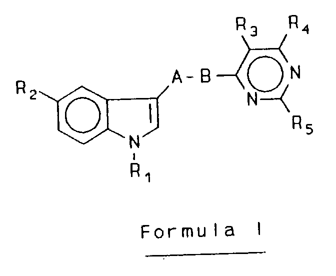
where R₁, R₂, R₃, R₄ and R₅ are as defined hereinafter, A is an optionally substituted alkyl or alkenyl chain and B is a group containing 1 or 2 atoms of nitrogen, selected from among an optionally substituted piperazine, aminopyrrolidine, pyrrolidine amine, piperidine or ethylene diamine.

These compounds act on the serotonin receptors and particularly on the 5-HT_{1D} receptor, both on the subtype 5HT_{1Dα} and on the 5HT_{1Dβ}, making them useful for the treatment of migraine.

### Prior art reference

It has been suggested that the pain caused by migraine might be associated with an excessive dilation of certain brain vessels and included among the known migraine treatments is the administration of compounds having vasoconstricting properties.

More recently, Dowie et al. disclose in GB-B-2 124 210 indoles which are selective agonists at the 5HT₁-like receptors.

Likewise, patent DE 3527648 discloses similar products, one of which is sumatriptan, a compound which has proved itself to be useful in the treatment of migraine. 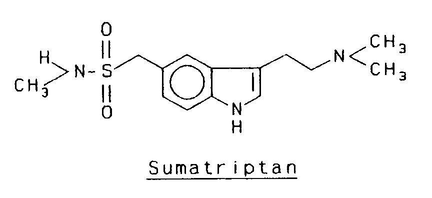
In the general Formula I, B may be a piperazine group, in which case the Formula I compounds may be indolalkylpiperazines.

A large number of indolalkylpiperazines have been described in the literature, some of which are claimed to have central nervous system depressing, antihypertensive, adrenolytic, antiinflammatory, antihemetic and muscular relaxing activities, among others. These compounds disclosed in the literature are disclosed, among others, in the following patents: US 3466287; US 3547922; US 3562278; US 13189064; US 3188313; US 944443; US 3888861; US 3888860; US 3466287; FR 1551082; FR 2102282; GB 1075156; EP 0548813; EP 0464558; EP 0464604.

Of all the products described in the literature, only Smith et al., in patents EP 0 464 558; EP 0 464 604 and EP 0 548 813, disclose substituted indolalkylpiperazines in which the substituent on the piperazine ring is a 4-pyrimidyl, in turn substituted.

When the B group of the general Formula I is a piperidine, these compounds correspond to a general formula of indolalkylpiperidines.

Very few indolalkylpiperidines have been disclosed in the literature (DE 3308668; GB 2044254; DE 2609289) and in no case are these compounds substituted in the piperidine ring with a pyrimidine heterocycle.

The same may be said where B is an aminopyrrolidine; a pyrrolidine amine or an ethylene diamine group (aminoethylamine).

In the case of the indolalkylpiperazines disclosed by Smith et al. in EP 0 464 558; EP 0 464 604 and EP 0 548 813, only a low molecular weight alkoxy group is claimed as substituent in the pyrimidine ring.

### Summary of the Invention

Research in connection with the invention has revealed that the substitution in this pyrimidine ring is very important for the activity, affinity and selectivity of the general Formula I compounds on the serotonin receptors and particularly on the 5-HT_{1D} (α and β) receptors and depending on the number of substituents, the relative position thereof and their chemical nature, the activity, affinity and selectivity on the 5-HT_{1D} (α and β) serotonin receptors vary enormously, ranging from compounds with very little affinity or with low selectivity to compounds having a great affinity and a high selectivity both for the 5-HT_{1Dα} serotonin receptors and for the 5-HT_{1Dβ} receptors, as is the case of the general Formula I compounds substituted in the pyrimidine ring, where R₁ and R₄ are low molecular weight alkoxy groups.

The invention claims the indole compounds of general Formula I, which have an affinity for the serotonin receptors and particularly are agonists and/or antagonists at the 5-HT_{1D} (α and/or β) receptor and are therefore applicable to disorders of the Central Nervous System and of the Peripheral Nervous System in which serotonin is involved, such as anxiety, depression, hypertension, psychotic processes, etc., and, in particular, the general Formula I products are applicable in the prevention and treatment of migraine.

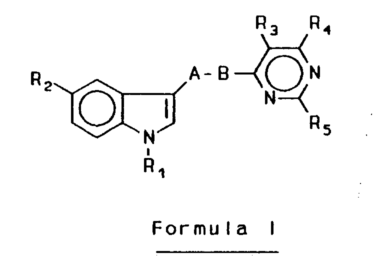

In general formula I:
- R₁: is an atom of hydrogen or a C₁₋₆ lower alkyl or C₁₋₆ lower acyl group;
- R₂: is an atom of hydrogen, halogen or a C₁₋₆ lower alkyl group, hydroxy, C₁₋₆ lower alkoxy, cyano, carboxamido, carboxy, alkoxycarbonyl, phenyl, or a -(CH₂)ₙ-R₆ group where n is 1, 2 or 3 and R₆ is cyano, nitro, carboxy, -CO₂R₇; -CONR₇; -SO₂NR₇R₈; -COR₇; -SO₂R₇, where R₇ and R₈ are independently an atom of hydrogen or a C₁₋₆ lower alkyl group,
R₇ and R₈ together with the N may also form a ring, having 4 to 7 members with one or more hetero atoms; said ring being optionally substituted by an alkyl group, hydroxy, alkoxy, alkoxycarbonyl, cyano, nitro, carboxamido, sulfonamido, phenyloxy, benzyloxy;
- R₃, R₄ and R₅: are independent of each other and are an atom of hydrogen, or halogen or a C₁₋₆ lower alkyl group, hydroxy, C₁₋₆ lower alkoxy; optionally substituted phenyl; optionally substituted phenyloxy; benzyloxy optionally substituted in the phenyl ring; NR₁₁R₁₂, where R₁₁ and R₁₂ are independent of each other and are an atom of hydrogen or a C₁₋₆ lower alkyl group, cyano, nitro, carboxy, alkoxycarbonyl, carboxamido or a -(CH₂)ₙ-R₆, where n and R₆ are as hereinbefore defined;
- R₃: nevertheless cannot be hydrogen or lower alkoxy when R₄ and R₅ are hydrogen and B is a piperazine ring;
- A: is either -(CH₂)ₘ- alkylidene group optionally substituted with hydroxy, m being an integer ranging from 1 to 5, or a C₁₋₅ alkenyl group;
- B: is a piperazine ring 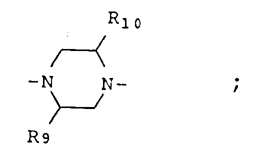 an aminopyrrolidine ring 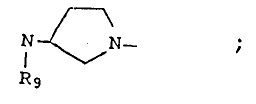 a pyrrolidineamino ring 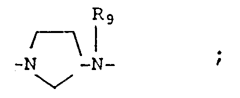 a piperidine ring 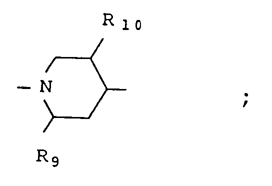 or ethylenediamino -NR₉-CH₂-CH₂-NR₁₀-
where R₉ and R₁₀
are independent of each other and are an atom of hydrogen or a C₁₋₆ lower alkyl group.

The invention also comprises the physiologically acceptable salts and solvates and salts of the solvates of the Formula I compounds and which include the acid addition salts formed with inorganic and organic acids, for example, hydrochlorides, hydrobromides, sulfates, nitrates, phosphates, formates, mesylates, citrates, benzoates, fumarates, maleates, lactates and succinates. When a salt of a Formula I compound is formed with a dicarboxylic acid, such as succinic acid, the salt may contain from one to two moles of the Formula I compound per mole of acid.

The preferred salts are the hydrochlorides and the succinates. The preferred solvates are the hydrates.

The Formula I compounds also additionally comprise the geometric isomers Z and E when A is an alkenyl group, as well as the stereoisomers and optical isomers such as for example the mixtures of enantiomers, as well as the individual enantiomers or the diastereoisomers resulting from the structural asymmetry existing in certain compounds of the present invention.

A preferred class of compounds represented by the Formula I are those where R₁ is an atom of hydrogen or a lower alkyl group;
- R₂: is -CH₂-SO2NR₇R₈, where R₇ and R₈ are independent of each other and are a C₁₋₆ lower alkyl group;
- A:: is a propenyl group -(CH₂)₃-;
- B:: is a piperazine ring or an ethylenediamine group -NR₉-CH₂-CH₂-NR₁₀, are independent of each other and are an atom of hydrogen or a C₁₋₆ lower alkyl group;
- R₃:: is a C₁₋₆ lower alkoxy group or an optionally substituted phenyl ring; and
- R₄:: is a C₁₋₆ lower alkoxy group.

A preferred compound in accordance with the present invention is C-(3-{3-[4-(5,6-dimethoxy-pyrimidin-4-yl)-piperazin-1-yl]-propyl}-1H-indol-5-yl)-N-methylmethanesulfonamide and the physiologically acceptable salts and solvates thereof.

The compounds of the present invention are useful in the treatment of conditions associated with headaches such as cluster headache, chronic paroxysmal hemicrania, pain associated with vascular disorders, headache associated with the administration or withdrawal of substances (e.g. withdrawal of drugs), headaches caused by tension and, particularly, they are useful for the treatment of migraine.

The treatments may be prophylactic or curative and are carried out by the administration, by any conventional way of administration, of a Formula I compound or a physiologically acceptable salt or solvate thereof.

More particularly, the invention relates to the indole derivatives having the following chemical names:
C-(3-{3-[1-(5-methoxy-pyrimidin-4-yl)-pyrrolidin-3-yl-amino]-propyl}-1H-indol-5-yl)-N-methylmethanesulfonamide
C-(3-{3-[1-(5,6-dimethoxy-pyrimidin-4-yl)-pyrrolidin-3-yl-amino]-propyl}-1H-indol-5-yl)-N-methylmethanesulfonamide
C-(3-{3-[3-(5-methoxy-pyrimidin-4-yl-amino)-pyrrolidin-1-yl]-propyl}-1H-indol-5-yl)-N-methylmethanesulfonamide
C-(3-{3-[3-(5,6-dimethoxy-pyrimidin-4-yl-amino)-pyrrolidin-1-yl]-propyl}-1H-indol-5-yl)-N-methylmethanesulfonamide
C-[3-(3-{2-[(5-methoxy-pyrimidin-4-yl)-methyl-amino]-ethylamino}-propyl)-1H-indol-5-yl]-N-methylmethanesulfonamide
C-[3-(3-{2-[(5,6-dimethoxy-pyrimidin-4-yl)-methyl-amino]-ethylamino}-propyl)-1H-indol-5-yl]-N-methylmethanesulfonamide
C-{3-[3-({2-[(5-methoxy-pyrimidin-4-yl)-methylamino]-ethyl}-methylamino)-propyl]-1H-indol-5-yl}-N-methylmethanesulfona mide
C-{3-[3-({2-[(5,6-dimethoxy-pyrimidin-4-yl)-methylamino]-ethyl}-methylamino)-propyl]-1H-indol-5-yl}-N-methylmethanesulfonamide
C-[3-(3-{2-[(5,6-dimethoxy-pyrimidin-4-yl)-amino]-ethylamino}-propyl)-1H-indol-5-yl}-N-methylmethanesulfonamide
C-(3-{3-[4-(6-methylamino-5-methoxy-pyrimidin-4-yl)-piperazin-1-yl]-propyl}-1H-indol-5-yl)-N-methylmethanesulfonamide
3-{3-[4-(5,6-dimethoxy-pyrimidin-4-yl)-piperazin-1-yl]-propyl}-5-fluoro-1H-indole
3-{3-[4-(5,6-dimethoxy-pyrimidin-4-yl)-piperazin-1-yl]-propyl}-5-methoxy-1H-indole
C-(3-{3-[3-(5,6-dimethoxy-pyrimidin-4-yl)-piperazin-1-yl]-1-methyl}-1H-indol-5-yl)-N-methylmethanesulfonamide
C-(3-{3-[3-(5,6-dimethoxy-pyrimidin-4-yl)-piperazin-1-yl]-1-methyl}-1H-indol-5-yl)-N,N-dimethylmethanesulfonamide
C-(3-{3-[4-(5-phenyl-pyrimidin-4-yl)-piperazin-1-yl]-propyl}-1H-indol-5-yl)-N-methylmethanesulfonamide
In accordance therewith, the invention also provides a pharmaceutical composition adapted for use in medicine, comprising: (a) a pharmaceutically effective amount of a Formula I compound and/or a salt or solvate thereof; and (b) a pharmaceutically acceptable excipient for oral, sub-lingual, parenteral, rectal or intranasal administration, or in a form appropriate for administration by inhalation or insufflation.

The invention also relates to the use of an indole derivative of Formula I for the preparation of a therapeutically applicable antimigraine drug.

The pharmaceutical compositions for oral administration may be solid, such as for example tablets or capsules prepared by conventional means with pharmaceutically acceptable excipients, or liquid such as for example aqueous or oily solutions, syrups, elixirs, emulsions or suspensions prepared by conventional means with pharmaceutically acceptable additives.

The compounds of the invention may be administered, if desired, in combination with one or more different therapeutical agents, such as analgesics, anti inflammatory agents and agents for nauseas.

The Formula I compounds and the physiologically acceptable salts and solvates thereof may be prepared by adaptation of the general methods explained hereinafter: 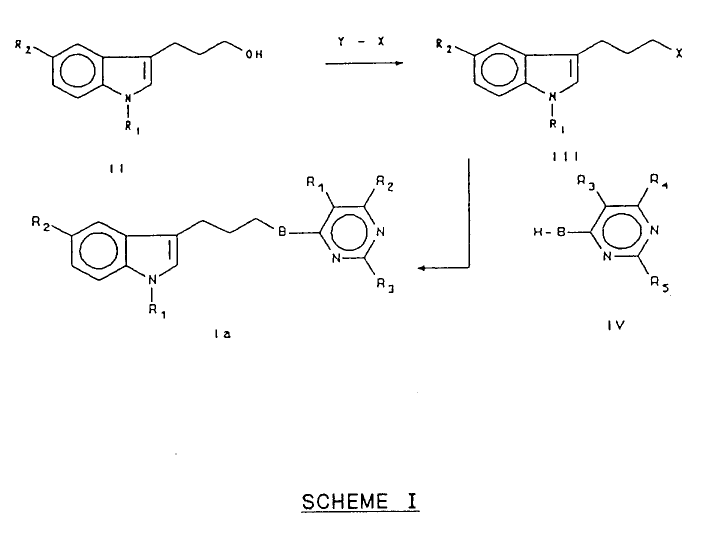
In the processes according to Scheme I, R₁ to R₅ and B are as defined hereinbefore. The reagent Y-X represents an organic leaving group where X is the leaving group fragment and may be tosyl, mesyl, halide, sulfate, phosphate, etc and Y is a proton or a counter ion: e.g. Y-X may be HBr, p-toluenesulfonyl chloride, etc.

According to scheme I, an indolalkylalcohol of Formula II is converted into an activated intermediate of Formula III in which the alcoholic moiety of the molecule is converted into an organic leaving group. Reaction of the intermediates III with the intermediate amines IV leads to the Formula I compounds where the group A defined in the general Formula is a propylene group.

The intermediate amines IV may be prepared by any of the general processes described in the literature (Smith D.W. et al., EP-A 0548813) consisting of the displacement, as detailed in Scheme II, of a halogen atom from the 4 position of the piperidine ring of the compounds V by an amine of the H-B-H type where B may be, as said above, a piperazine, aminopyrrolidine, pyrrolidineamine or ethylenediamine group. 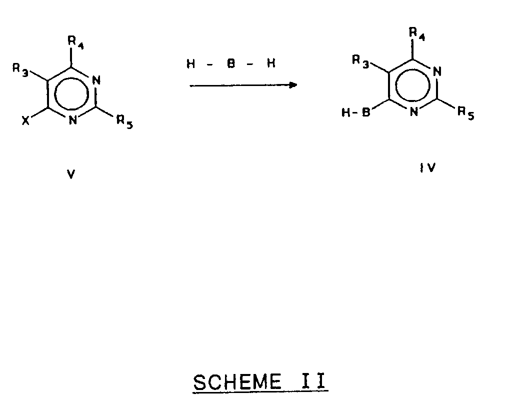
The Formula Ia compounds may also be prepared by a process similar to the one described in Scheme II for the preparation of the intermediates IV. In this case, as shown in Scheme III, 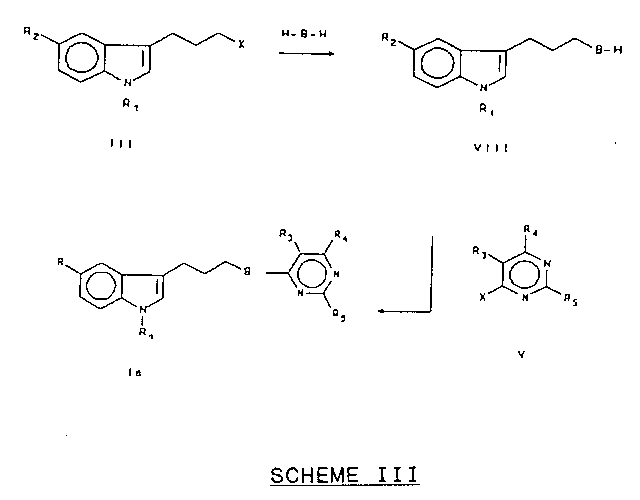
The process consists of reacting a Formula III compound where R₁, R₂ and X are as hereinbefore defined with an amine H-B-H where B is as hereinbefore defined thereby to produce an intermediate VIII which, by subsequent reaction with an intermediate V where X is a halogen atom and R₃, R₄ and R₅ are as hereinbefore defined, leads to the products of the general Formula Ia where the group A defined in the general Formula I is a propylene group.

Another process for preparing the Formula I compounds is as shown in Scheme IV: 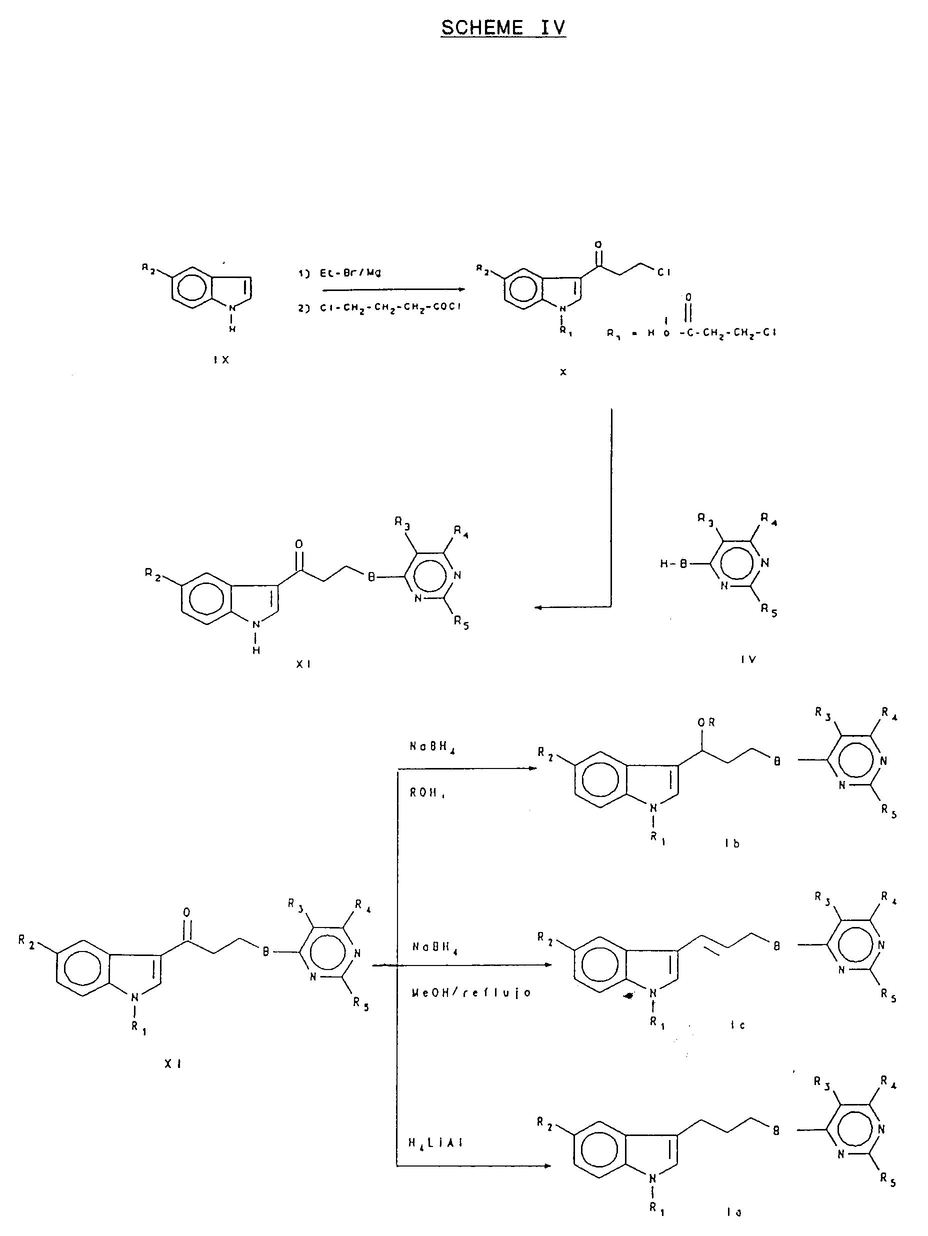
According to this process, an indole of Formula IX is acylated with 3-chloropropionyl chloride as per the methods described in the literature (See, e.g. F. Troxler in Helv. Chiar Acta 42, 2073 (1959) or Marchese G.P. in Chim. Ind. (Milan) 51 (3), 258-262 (1969)) thereby to prepare a compound of Formula X which by displacement of the chlorine atom of the side chain from the 3 position of the indole by a type IV amine hereinbefore described leads to the preparation of the general Formula XI compounds where R₁, R₂, R₃, R₄, R₅ and B are as hereinbefore defined. These products of the general Formula XI may be converted by different known processes into products of the general Formula I where A is a propylene group (Ia) or a propylene group substituted with a hydroxy or alkoxy group (Ib) or A is a propenylene group (Ic).

Another alternative process for preparing general Formula I products (Scheme V) consists, as described in ES 2033577 and ES 2033578, in reacting an indole of general Formula XII 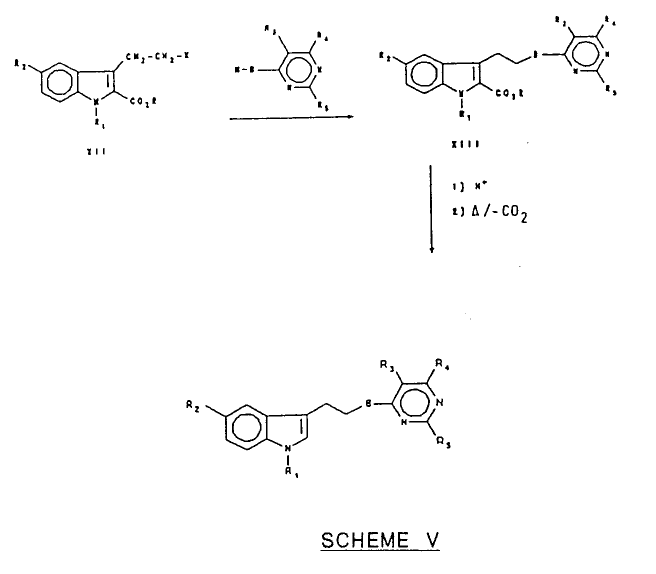
where X is a leaving group such as, e.g., p-toluenesulfonyl and R is an alkyl group, with a type IV amine thereby to prepare the compounds XIII, which are subjected to a process of saponification of the ester group in the 2 position of the indole ring and in a second step to decarboxylation of the acid obtained, thereby leading to the compounds Id where the group A defined in the general Formula I is an ethylene group.

Finally, the general Formula I products may be prepared as described in Scheme VI by processes consisting of modifying some of the substituents of general Formula I with another substituent, according to methods known in the literature.

Thus, for example, when in general Formula I products R₁ is hydrogen, they may be converted into general Formula I products where R₁ is alkyl or acyl by alkylation or acylation with an X-R reagent, where X is a good leaving group such as halogen, tosyl, mesyl, sulfate, etc. 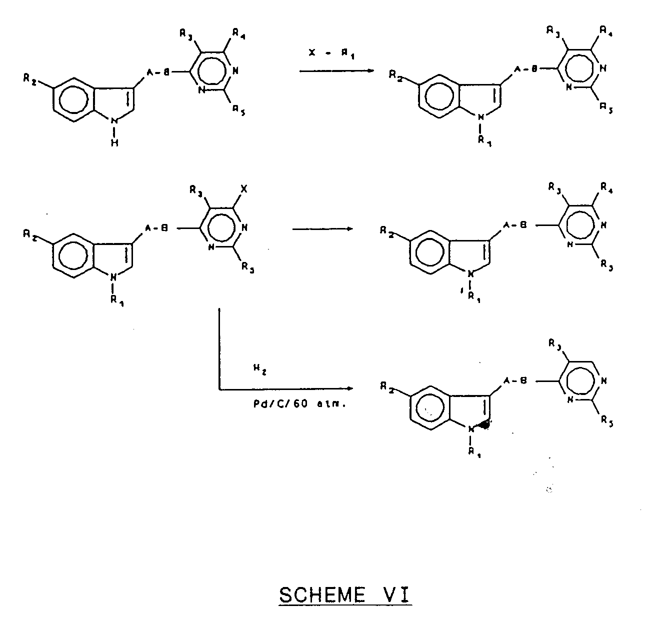
Likewise, in the Formula I products where R₄ is a halogen atom, this may be substituted by an alkoxy or by an amine by refluxing the product I in the appropriate solvent in presence of an alkylate or an amine.

The halogen atom in R₄ may also be substituted by an atom of hydrogen through a hydrogenolysis carried out preferably in the presence of a Pd catalyst at a pressure ranging from 20 to 60 atm.

These conversions, in which R₄ is a halogen atom substituted by an alkoxy or amino group or by a hydrogen atom, may be carried out also on the general Formula IV intermediates thereby to produce intermediates of the same general formula where R₄ is alkoxy, amino or hydrogen, respectively.

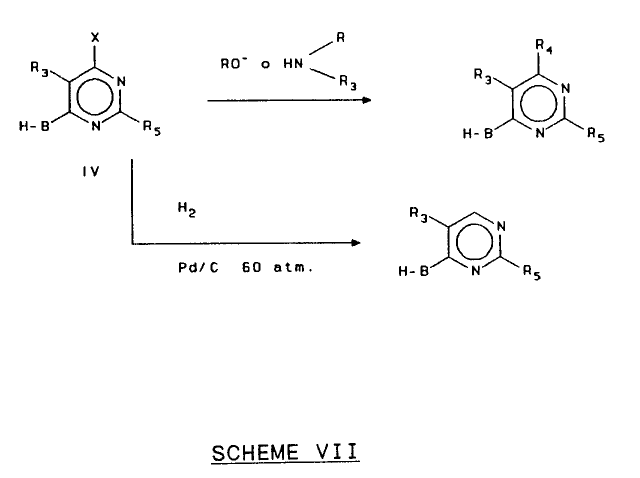

### FORMULA V PRODUCTS

### Example 1

### 4,6-dihydroxy-5-methoxypyrimidine

In like manner to that described by Smith D.W. et al., EP 0548813, 175 g (3.24 mol) of sodium methoxide were dissolved in 1.0 L of absolute methanol. The mixture was cooled to 10°C and 162.1 g (1.0 mol) of dimethylmethoxymalonate and 104.1 g (1.0 mol) of formamidine acetate were added. The mixture was stirred for 30 minutes at 0°C and afterwards for 1 hour at reflux. It was cooled in an ice-water bath and 350 ml of concentrated hydrochloric acid were added. The precipitate formed was filtered and suspended in 400 ml of water at 10°C and filtered again. The white solid obtained was dried in vacuo, yielding 115.7 g (81%).

### Example 2

### 4-hydroxy-2-methyl-5-methoxypyrimidine

A modified form of the Maccoss Malcolm EP 330263 process was used.

To a suspension of sodium (4.88 g 0.212 at-gr) in 300 ml of ethyl ether was added dropwise over 1 hour a mixture of 25 g (0.212 mol) of ethylmethoxyacetate and 23.5 g (0.23 mol) of ethyl formate. At the end of the addition, the reaction was refluxed under a N₂ atmosphere for 2 hours and thereafter at room temperature overnight. The solvent was removed by decantation and the residue was washed with 3 x 100 ml of ethyl ether. The residue was dissolved in 250 ml of ethanol and 20 g (0.212 mol) acetamidine chloride were added. The mixture was refluxed for 6 hours. It was cooled to room temperature and the salts were filtered. The filter liquors were concentrated to dryness and chromatographed on silica gel, eluting with dichloromethane/ethanol 7/3, yielding 16.7 g of a white solid (56.2%).

### Example 3

### 4,6-dichloro-5-methoxypyrimidine

A modified form of the process described by Bretschneider, Richter and Klötze, Monatsh. Chem. 96 (6), 1661-76 (1965) was used.

A mixture of 4,6-dihydroxy-5-methoxypyrimidine (113.6 g 0.8 mol), POCl₃ (600 ml) and N,N-diethylaniline (50 ml) was heated to reflux for 3 hours. The excess phosphorous oxychloride was removed by distillation in vacuo. The residue was poured over ice-water. The white solid formed was filtered. It was dissolved in heptane-ethyl acetate 7/3 (400 ml) and chromatographed on silica gel, eluting with Hep/AcOEt 7/3, thereby yielding 108.2 g of a white solid (75.6%).

### Example 4

### 2,4-dichloropyrimidine

Operating in a like manner to above and starting out from uracil (11.2 g 0.1 mol), 10.5 g (70.5%) of a white solid were yielded.

### Example 5

### 2,4-dichloro-5-methylpyrimidine

Operating as described above and starting out from 12.6 g (0.1 mol) of thymine, 12.4 g (70%) were yielded.

### Example 6

### 4-chloro-2-methyl-5-methoxypyrimidine

Operating as described above and starting out from 14 g (0.1 mol) of 4-hydroxy-2-methyl-5-methoxy pyrimidine, 12.3 g (69.1%) of product in form of a white solid were yielded.

### Example 7

### (6-chloro-5-methoxy-pyrimidin-4-yl)-methylamine

To 17.9 g (0.1 mol) of 4,6-dichloro-5-methoxypyrimidine in 100 ml of absolute ethanol were added 31.25 ml (0.25 mol) of an 8M solution of methylamine in ethanol. The reaction was maintained with stirring, the temperature being controlled so as not to rise above 30°C. At the end of 1 hour, 100 ml of a solution of 1N NaOH were added. The reaction mixture was concentrated to dryness. The residue was treated with 100 ml of distilled water and extracted with 3 x 100 ml of CH₂Cl₂. The extracts were dried and concentrated to yield 13.4 g (92%) of a white solid.
¹H-NMR: (DMSO-d6, ppm): 8.12 (s, 1H), 7.67 (s.a.), 3.80 (s, 3H), 2.95 (d, 3H)

### Example 8

### 4-chloro-5,6-dimethoxypyrimidine

Following a modified form of the process described by Bretschneider et al., Monatsh Chem. 96, 1661-1669 (1965), to 6 g (0.11 mol) of sodium methoxide in 150 ml of methanol were added at 0°C 17.9 g (0.1 mol) of 4,6-dichloro-5-methoxypyrimidine. The mixture was held at 0°C with stirring for 1 hour. It was allowed to rise to room temperature (25°C), the salts formed were filtered. The filter liquors were concentrated to dryness. The residue was treated with 100 ml of distilled water and extracted with 3 x 100 ml of CH₂Cl₂. The extracts were dried and concentrated, thereby yielding 16.1 g of a completely pure white solid. (92.2%).
¹H-NMR: (DMSO-d6, ppm): 8.41 (s, 1H); 4.03 (s, 3H) 3.88 (s, 3H)

### FORMULA IV PRODUCTS

### Example 9

### 4-chloro-5-methoxy-6-piperazin-1-yl-pyrimidine

A modification of the process used by Smith et al. in EP 0464604 was used.

To a solution of 21.5 g (0.25 mol) of piperazine in 150 ml of EtOH and 50 ml of water stirred vigorously at 25°C were added 17.9 g (0.1 mol) of 4,6-dichloro-5-methoxypyrimidine. The reaction was held for 3 hours, with the temperature held to between 25-30°C. At the end of this time, the reaction mixture was concentrated to dryness and to the residue was added 100 ml of a solution 1N NaOH followed by extraction with 3 x 100 ml of dichloromethane. The organic phase was dried and concentrated. The residue was purified by chromatography on silica gel, eluting with CH₂Cl₂/EtOH/NH₄OH 70/25/5 thus to yield 19.2 g (83.9%) of a colourless oil which rapidly solidifies.

### Example 10

### N1-(6-chloro-5-methoxy-pyrimidin-4-yl)-N1,N 2-dimethylethane-1,2-diamine

Following the process described in Example 9 and starting out from 17.9 g (0.1 mol) of 4,6-dichloro-5-methoxypyrimidine and 22 g (0.25 mol) de N,N'-dimethyl ethylenediamine (Aldrich D15, 780-5), 20.6 g (89.3%) of an oil were yielded.
¹H-NMR: (DMSO-d₆ ppm): 8.10 (s, 1H); 3.70 (t, 2H); 3.68 (s, 3H); 3.22 (s, 3H); 2.69 (t, 2H) 2.62 (s.a, 1H); 2.28 (s., 3H).

### Example 11

### N1-(6-chloro-5-methoxy-pyrimidin-4-yl)-N1-methyl-ethane-1,2-diamine

Following the process described in Example 9 and starting out from 17.9 g (0.1 mol) of 4,6-dichloro-5-methoxypyrimidine and 11.1 g (0.15 mol) of N-methylethylenediamine, 13.6 g (62.8%) of an oil were yielded.
¹H-NMR: (DMSO-d6, ppm): 8.1 (s, 1H); 368 (s, 3H) 3.62 (t, 2H); 3.21 (s, 3H); 2.75 (t, 2H); 1.5 (s.a, 2H)

### Example 12

### N1-(6-chloro-5-methoxy-pyrimidin-4-yl)ethane-1,2-diamine

Following the process described in Example 9 and replacing the piperazine with 15 g (0.25 mol) of ethylenediamine (Aldrich 12, 701-9), 16.2 g (79.9%) of an oil which rapidly crystallises were yielded.
¹H-NMR: (DMSO-d6, ppm): 8.06 (s, 1H) 7.6 (s.a, 1H); 3.75 (s, 3H); 3.42-3.30 (m, 2H) 2.72 (t, 2H); 1.7 (s.a, 2H)

### Example 13

### 4-chloro-5-methoxy-6-(3-methyl-piperazin-1-yl)-pyrimidine

Following the process described in Example 9 and replacing the piperazine with 2-methylpiperazine (Aldrich M7, 240-4) (15 g, 0.15 mol) 23 g (94.8%) of a white solid were yielded.
¹H-NMR: (D₂O ppm): 8.2 (s, 1H); 4.65 (d, 2H) 3.82 (s, 3H); 3.65-3.22 (s.c, 5H) 1.43 (d, 3H).

### Example 14

### 4-(6-chloro-5-methoxy-pyrimidin-4-yl)-piperazin-1-carbaldehyde

17.9 g (0.1 mol) of 4,6-dichloro-5-methoxypyrimidine, 11.4 g (0.1 mol) of 1-piperazinylcarboxaldehyde and 13.8 g (0.1 mol) of potassium carbonate in 150 ml of acetonitrile were stirred at 20°C for 48 hours. The reaction mixture was concentrated to dryness, 100 ml of distilled water were added, followed by extraction with con 3 x 100 ml of CH₂Cl₂. The extracts were dried and concentrated. 24.3 g (94.7%) of a white solid were yielded.
¹H-NMR: (CDCl₃, ppm): 8.22 (s, 1H); 8.12 (s, 1H) 3.95-3.82 (s.c., 4H); 3.79 (s, 3H); 3.65 (s.c, 2H); 3.50 (s.c., 2H)

### Example 15

### 1-(6-chloro-5-methoxy-pyrimidin-4-yl)-pyrrolidin-3-yl-amine

Following the process described in Example 9, to 17.9 g (0.1 mol) of 4,6-dichloro-5-methoxypyrimidine in 150 ml of ethanol and 50 ml of water was added an aqueous solution of 3-aminopyrrolidine (0.3 mol) obtained by neutralization of 47.7 g 3-aminopyrrolidine dihydrochloride with 60 ml of 5N NaOH. After 12 hours, the reaction was alkalized with 1N NaOH (100 ml) and was concentrated in the rotary evaporator until the 150 ml of ethanol had been removed. The resulting aqueous solution was extracted with 3 x 100 ml of CH₂Cl₂. The extracts were dried and concentrated, yielding 21.4 g (94%) of a white solid used without further purification.
¹H-NMR: (CDCl₃, ppm): 8.1 (s, 1H); 3.95-3.62 (s.c, 4H); 3.78 (s, 3H); 3.44 (dd, 1H) 2.24-2.08 (m, 1H); 1.86-1.70 (m, 1H) 1.72 (s.a, 2H)

### Example 16

### (1-benzyl-pyrrolidin-3-yl)-(6-chloro-5-methoxy-pyrimidin-4-yl)amine

### 1-benzylpyrrolidin-3-yl-amine

To a solution of 32 g (0.2 mol) of 3-aminopyrrolidine dihydrochloride in 50 ml of water were added 80 ml of a solution of 5N NaOH (0.4 mol). To the resulting solution were added 25.3 g (0.2 ml) of benzyl chloride in 150 ml of ethanol. The reaction was heated to 80°C for 6 hours. It was cooled to room temperature. 40 ml of 5N NaOH were added. The mixture was concentrated to dryness and the residue was chromatographed over silica gel, eluting with CH₂Cl₂/EtOH/NH₄OH 70/25/5, thereby yielding 26 g (73.9%) of a liquid.

### (1-benzyl-pyrrolidin-3-yl)-(6-chloro-5-methoxy-pyrimidin-4-yl)amine

24.6 g (0.14 mol) of 3-amino-1-benzylpyrrolidine obtained in the previous step and 17.9 g (0.1 mol) of 4,6-dichloro-5-methoxypyrimidine were heated to reflux in 300 ml of acetonitrile for 6 hours and at room temperature overnight. The mixture was concentrated to dryness and 100 ml of 1N NaOH were added, followed by extraction with 3 x 100 ml of dichloromethane. The extracts were washed with 100 ml of a saturated solution of sodium chloride. The organic phase was dried and concentrated to dryness. The residue obtained was chromatographed on silica gel, eluting with CH₂Cl₂/EtOH 95/5, yielding 18.7 g (64.3%) of product.
¹H-NMR: (CDCl₃, ppm): 8.12 (s, 1H); 7.53-7.20 (s.c., 5H); 5.60 (d, 1H); 4.70-4.55 (m, 1H); 3.85 (s, 3H); 3.64 (s, 2H); 2.95-2.85 (m, 1H); 2.78-2.60 (s.c., 2H); 2.44-2.30 (s.c., 2H); 1.80-1.60 (m, 1H)

### Example 17

### 2-chloro-4-piperazin-1-yl-pyrimidine

Following the process described in Example 9 and starting out from 7.45 g (0.05 mol) of 2,4-dichloropyrimidine and 21.5 g (0.25 mol) of piperazine, 8.4 g (84.6%) of a white solid were yielded.
¹H-NMR: (TFA-d1, ppm): 8.20 (d, 1H); 4.7-3.7 (s.c., 8H)

### Example 18

### 5,6-dimethoxy-4-piperazin-1-yl-pyrimidine

### Method A

12.8 g (0.05 mol) of 4-(6-chloro-5-methoxy-pyrimidin-4-yl)-piperazin-1-carbaldehyde (Example 14) and 6.5 g (0.12 mol of sodium methoxide were heated to reflux in 50 ml of absolute methanol for 14 hours. The reaction mixture was concentrated to dryness. 50 ml of distilled water were added, followed by extraction with 4 x 50 ml of CH₂Cl₂. The organic phase was dried and concentrated. The residue was chromatographed over silica gel, eluting with CH₂Cl₂/EtOH/NH₄OH 70/25/5. 7.6 g (67.8%) of a white solid were thus yielded.
¹H-NMR: (CDCl₃, ppm): 8.10 (s, 1H); 4.00 (s, 3H) 3.72 (t, 4H); 3.71 (s, 3H); 2.97 (t, 4H) 1.92 (s, 1H)

### Method B

Starting out from 4-chloro-5-methoxy-6-piperazin-1-yl-pyrimidine (11.4 g, 0.05 ml) and following the same method, 9.1 g (81.5%) of product were yielded.

### Method C

8.73 g (0.05 mol) of 4-chloro-5,6-dimethoxy-pyrimidine and 8.6 g (0.1 mol) of piperazine were heated to reflux in 50 ml of absolute ethanol for 5 hours. The mixture was concentrated to dryness and 50 ml of 1N NaOH were added, followed by extraction with 3 x 50 ml of CH₂Cl₂. The extracts were dried and concentrated. The residue was chromatographed over silica gel, eluting with CH₂Cl₂/EtOH/NH₄OH 70/25/5. 9.8 g (87.4%) were yielded.

### Example 19

### N1-(5,6-dimethoxy-pyrimidin-4-yl)-N1,N2-dimethyl-ethane-1,2-diamine.

To 6.9 g (0.03 mol) of N1-(6-chloro-5-methoxy-pyrimidin-4-yl)-N 1,N 2-dimethyl-ethane-1,2-diamine (Example 10) in 50 ml of absolute methanol were added 9.6 g (0.15 mol) of sodium methoxide and the reaction was refluxed for 12 hours. The mixture was concentrated to dryness, 50 ml of distilled water were added and it was extracted with 5 x 50 ml of dichloromethane. The organic phase was dried and concentrated and the residue was chromatographed over silica gel, eluting with CH₂Cl₂/EtOH/NH₄OH 70/25/5, thereby yielding 5.6 g (82.5%) of a white solid.
¹H-NMR: (DMSO-d6, ppm): 7.96 (s, 1H); 3.85 (s, 3H); 3.60 (s, 3H); 3.59 3,(t, 2H); 3.12 (s, 3H) 2.65 (t, 2H); 2.26 (s, 3H); 1.75 (s.a, 1H)

Following the same process as described in the previous Example and starting out from the corresponding aminopyrimidine (Examples 11-12, 15-16, 7), the following products were yielded:

### Example 20:

### N1-(5,6-dimethoxy-pyrimidin-4-yl)-N1-methyl-ethane-1,2-diamine

¹H-NMR: (DMSO-d6, ppm): 7.98 (s, 1H); 3.85 (s, 3H) 3.60 (s, 3H); 3.52 (t, 2H); 3.11 (s, 3H); 2.72 (t, 2H) 1.40 (s.a, 2H).

### Example 21:

### N1-(5,6-dimethoxy-pyrimidin-4-yl)-ethane-1,2-diamine

¹H-NMR: (DMSO-d6, ppm): 1.95 (s, 1H); 6.82 (t, 1H) 3.88 (s, 3H); 3.65 (s, 3H); 3.32 (dt, 2H) 2.68 (t, 2H); 1.40 (s.a., 2H)

### Example 22:

### 1-(5,6-dimethoxy-pyrimidin-4-yl)pyrrolidin-3-yl-amine

¹H-NMR: (CDCl₃, ppm): 8.08 (s, 1H); 3.98 (s, 3H) 3.94-3.60 (s.c., 4H); 3.70 (s, 3H) 3.46-3.36 (m, 1H); 2.20-2.04 (m, 1H) 1.82-1.70 (m, 1H); 1.12 (s.a., 2H)

### Example 23:

### (1-benzyl-pyrrolidin-3-yl)-(5,6-dimethoxy-pyrimidin-4-yl)amine

¹H-NMR: (CDCl₃, ppm): 8.06 (s, 1H); 7.32-7.22 (s.c., 5H) 5.39 (d, 1H) 4.6 (m, 1H); 3.95 (s, 3H) 3.79 (s, 3H); 3.62 (s, 2H); 2.90-2.56 (s.c., 3H); 2.42-2.30 (m, 2H); 1.68-1.58 (m, 1H)

### Example 24:

### (5-methoxy-6-piperazin-1-yl-pyrimidin-4-yl)-methylamine

¹H-NMR: (DMSO-d6, ppm): 7.88 (s, 1H); 6.60 (q, 1H) 3.52 (s, 3H); 3.38 (t, 4H); 3.10 (s.a., 1H) 2.80 (d, 3H); 2.75 (t, 4H)

### Example 25:

### 1-(5,6-dimethoxy-pyrimidin-4-yl),N-(pyrrolidin-3-yl)-amine

To 6.3 g (0.02 mol) of (1-benzyl-pyrrolidin-3-yl)-(5,6-dimethoxy-pyrimidin-4-yl)amine in 50 ml of ethanol were added 0.6 g of 10% Pd/C, followed by hydrogenation at atmospheric pressure for 48 hours. The catalyst was filtered out. The filter liquors were concentrated to dryness and the residue was chromatographed over silica gel, eluting with CH₂Cl₂/EtOH/NH₄OH 70/25/5, thereby yielding 4.2 g (93.6%) of a white solid.
¹H-NMR: (DMSO-d6, ppm): 7.98 (s,1H); 6.66 (d, 1H): 4.48-4.32 (m, 1H); 3.88 (s, 3H); 3.65 (s, 3H) 2.98-2.84 (s.c., 2H); 2.78-2.60 (s.c., 2H) 2.08-1.90 (s.c., 1H); 1.70-1.55 (s.c., 1H)

### Example 26

### N1-(5-methoxy-pyrimidin-4-yl)-N1,N2-dimethyl-ethane-1,2-diamine

To 11.4 g (0.05 mol) of N-1-(6-chloro-5-methoxy-pyrimidin-4-yl)-N1,N2-dimethylethane-1,2-diamine (Example 10) in 50 ml of ethanol were added 10 ml of 5N HCl and 1.1 g of 10% Pd/C. The mixture was hydrogenated at 60 atm and 25°C for 12 hours. It was filtered over decalite and the decalite was washed with water. The filter liquors were concentrated to dryness. The residue was treated with 50 ml of 2N NaOH and extracted with 4 x 50 ml of CH₂CL₂. The extracts were dried and concentrated to leave an oil which crystallized on standing and was used without further purification. 7.6 g (77.5%) were yielded.
¹H-NMR: (DMSO-d6): 8.20 (s, 1H); 7.94 (s, 1H) 3.84 (s, 3H); 3.68 (t, 2H), 3.18 (s, 3H) 2.70 (t, 2H); 2.30 (s, 3H); 2.10 (s.a., 1H)
Following the same process, the following compounds were yielded:

### Example 27:

### N1-(5-methoxy-pyrimidin-4-yl)-N1-methyl-ethane-1,2-diamine

¹H-NMR: (DMSO-d6, ppm): 8.12 (s, 1H); 7.90 (s, 1H) 3.80 (s, 3H); 3.58 (t, 2H); 3.12 (s, 3H); 2.71 (t, 2H); 1.4 (s.a., 2H)

### Example 28:

### 1-(5-methoxy-pyrimidin-4-yl)-pyrrolidin-3-ylamine

¹H-NMR: (CDCl₃, ppm): 8.22 (s, 1H) 7.75 (s, 1H) 3.99-3.85 (s.c., 2H); 3.84-3.72 (m, 1H) 3.80 (s, 3H); 3.68-3.60 (m, 1H); 3.52-3.46 (m, 1H); 2.20-2.04 (m, 1H); 1.80-1.60 (m, 1H) 1.40 (s.a., 2H).

### Example 29

### N-(5-methoxy-pyrimidin-4-yl),N-(pyrrolidin-3-yl)-amine

Starting out from 5.8 g (0.02 mol) of the product obtained in Example 16 and following the process described in Example 26 2.8 g (72%) of N-(5-methoxy-pyrimidin-4-yl), N-(pyrrolidin-3-yl)-amine were yielded.
¹H-NMR: (DMSO-D₂O, ppm): 8.09 (s, 1H); 7.72 (s, 1H); 4.42 (m, 1H); 3.82 (s, 3H) 3.08-2.90 (s.c., 2H); 2.84-2.72 (s.c., 2H) 2.18-2.00 (m, 1H): 1.76-1.60 (m, 1H).

### Example 30

### 4-chloro-5-phenyl-2-methylthio-6-piperazin-1-yl-pyrimidine

Following the process described in Example 9 and starting out from 13.56 g (0.05 mol) of 4,6-dichloro-5-phenyl-2-methylthio pyrimidine (Aldrich 14, 419-3) and 10.76 g (0.125 mol) of piperazine, 15.1 g (94%) of product in form of white solid were yielded.
¹HRM: 7.53-7.28 (m, 5H); 3.17-3.08 (m, 4H) 2.53-2.44 (m, 4H); 2.45 (S, 3H)

### Example 31

### 4-chloro-5-phenyl-6-piperazin-1-yl-pyrimidine

To a solution of 12.8 g (0.04 mol) of the product obtained in the previous Example in 300 ml of methanol and 150 ml of THF were added 66.4 g (0.28 mol) of nickel (II) hexahydrate chloride. The mixture was cooled to -20°C and 30.25 g (0.8 mol) of sodium borohydride were added slowly. At the end of the addition, the mixture was allowed to reach room temperature and was stirred for a further 3 hours. It was filtered over decalite. The decalite was washed with 50 ml of methanol and the filter liquors were concentrated to dryness and the residue was chromatographed over silica gel. Elution in CH₂Cl₂/EtOH/NH₄OH 75/25/5 yielded 2.1 g (19%) of a white solid.

### Example 32

### 5-phenyl-6-piperazin-1-yl-pyrimidine

When the chromatographic column of the previous reaction was eluted with CH₂Cl₂/EtOH/NH₄OH 75/25/5, 2.8 g (29%) of another product which was identified as 5-phenyl-6-piperazin-1-yl-pyrimidine were yielded.

### FORMULA II PRODUCTS

### Example 33

### 3-(5-fluoro-1H-indol-3-yl)-propan-1-ol

This product has been described previously by Smith et al. EP-A-0 464 558. We have synthesized this product using a process different from the one described by these workers. We used a modification of the process described by Demerson C.A. et al. for the synthesis of 1-cyclopropyltryptophol in J. Med. Chem. 19, 391-395 (1976).

To a solution of 90.8 g (0.558 mol) of 4-fluorophenylhydrazine hydrochloride in 50 ml of water and 500 ml of 2-methoxyethanol heated to 30°C were added dropwise 72.8 ml (0.8 mol) of dihydropyrane.

At the end of the addition, the reaction was heated to 85-90°C. The reaction was held under reflux for 15 min. It was allowed to cool to 20°C and the precipitated salts were filtered. 2L of ethyl ether were added and the precipitated salts were filtered again. The filter liquors were washed with 2 x 500 ml of distilled water. The ether extracts were dried and concentrated to dryness. The residue obtained was chromatographed over silica gel, eluting with dichloromethane, thereby yielding 105 g (97.4%) of an oil.
¹H-NMR: δ (200 MHz): 10.95 (s.a, 1H); 7.35 (d,d, 1H); 7.30 (d,d, 1H); 7.22 (d, 1H) 6.96-6.86 (s.c., 1H); 4.44 (s.a., 1H; 3.46 (t, 2H) 2.70 (t, 2H); 1.84-1.72 (m, 2H)

### Example 34

### 3-(5-methoxy-1H-indol-3-yl)-propan-1-ol

Following a process similar to the one described in Example 33 and starting out from 17.5 g (0.1 mol) of 4-methoxyphenylhydrazine hydrochloride, 8.5 g (41.5%) of an oil were yielded.

### Example 35

### C-[3-(3-hydroxypropyl)-1H-indol-5-yl]-N-methyl-methanesulfonamide

This product has been previously described by Smith D.W. et al. in EP-A-0548813. There is now described a different process to the one used by these workers, consisting of a modification of the Process described in Example 33.

To a solution of 151 g (0.6 mol) of N-methyl-4-methanesulfonamidophenylhydrazine hydrochloride in 1L of distilled water and 1L of ethanol cooled to 10°C were added dropwise over ½ hour 60 ml (0.66 mol) of freshly distilled dihydropyrane. At the end of the addition, the reaction was held at 20°C for 3 hours, with the formation of the intermediate hydrazone being observed by TLC (CH₂Cl₂/EtOH 9/1). At the end of this time, the reaction was heated to 60°C for 6 hours, and was afterwards held at room temperature overnight. It was concentrated in the rotary evaporator until the total volume of the reaction mixture had been reduced to 700 ml.

The resulting residue was treated with 3 x 300 ml of ethyl acetate. The ethyl acetate extracts were dried and concentrated and the residue obtained was chromatographed over silica gel, eluting with CH₂Cl₂/EtOH 9/1, thereby yielding 68.2 g (40.3%) of a solid.
¹H-NMR: δ (200 MHz, DMSO-d6): 10.82 (d, 1H); 7.52 (d, 1H); 7.33 (d, 1H); 7.14 (d, 1H) 7.08 (dd, 1H); 6.78 (q, 1H); 4.44 (t, 1H) 4.36 (s, 2H); 3.52-3.40 (m, 2H); 2.7 (t, 2H) 2.54 (d, 3H); 1.86-1.72 (m, 2H).

### FORMULA III PRODUCTS

### Example 36 General Process.

### 3-[5-[[(methylamino)sulfonyl]methyl]-1H-indol-3-yl]propyl-p-toluenesulfonate

To 68.2 g (0.241 mol) of C-[3-(3-hydroxypropyl)-1H-indol-5-yl]-N-methyl-methanesulfonamide, prepared as described in Example 35, in 400 ml of distilled pyridine and at 0°C were added 50.7 g (0.266 mol) of p-toluenesulfonyl chloride. The addition was effected portionwise, holding the temperature a 0°C. At the end of the addition (approx. 15 min) the reaction was allowed to rise to room temperature and was held with stirring for 3 hours. The reaction was poured over 1 L of water at 0°C and was acidulated with concentrated hydrochloric acid. It was extracted with 3 x 500 ml of dichloromethane. The extracts were washed with 200 ml of 1N HCl afterwards with 200 ml of 2N NaOH and finally with 2 x 300 ml of saturated sodium chloride solution.

The organic phase was dried with sodium sulfate, was filtered and was concentrated. The residue was chromatographed over silica gel eluting with CH₂Cl₂/EtOH 98/2, thereby yielding 50 g (47,4%) of a white solid.
¹H-NMR: δ (200 MHz, CDCl₃): 8.25 (s.a., 1H) 7.77 (d, 2H); 7.52 (s, 1H); 7.32 (d, 2H); 7.31 (d, 1H); 7.16 (d, 1H); 6.92 (s.a., 1H); 4.32 (s, 2H); 4.18 (q, 1H); 4.03 (t, 2H); 2.79 (t, 2H); 2.66 (d, 3H); 2.42 (s, 3H); 2.06-1.94 (m, 2H)

### Example 37

### 3-(5-methoxy-1H-indol-3-yl)-propyl-p-toluenesulfonate

Following the above process and starting out from 8.5 g (0.041 mol) of 5-methoxy-3-(3-hydroxypropyl)indole, 8 g of an oil (53.7%) were yielded.

### Example 38

### 3-(5-fluoro-1H-indol-3-yl)-propyl-p-toluenesulfonate

This product, which has already been prepared by Smith et al., as disclosed in EP-A-0464558, has now been prepared by the general process described in Example 36 from 105 g of 5-fluoro-3-(3-hydroxypropyl)indole. Subsequent purification in a column of silica gel, eluting with heptane/AcOEt 7/3 yielded 141.4 g (74.9%) of a solid.
¹H-NMR: δ (200MHz, DMSO-d6): 10.92 (s.a., 1H) 7.82 (d, 2H); 7.48 (d, 2H); 7.38 (dd, 1H) 7.21 (d, 1H); 7.14 (s, 1H); 6.94 (dd, 1H) 4.08 (t, 2H); 2.63 (t, 2H); 2.41 (s, 3H); 1.98-1.84 (m, 2H).

### FORMULA VIII PRODUCTS

### Example 39: General process

### 5-fluoro-3-(3-piperazin-1-yl-propyl)-1H-indole

To a solution of 3-(5-fluoro-1H-indol-3-yl)-propyl-p-toluenesulfonate (20.8 g, 0.06 mol) in 400 ml of acetonitrile were added 15.5 g (0.18 mol) of piperazine. The reaction was held under reflux for 2 hours. It was concentrated to dryness and the residue was treated with 170 ml of 1N NaOH and extracted with 3 x 100 ml of CH₂Cl₂. The extracts were dried with 2SO₄Na₂, were filtered and concentrated and the residue was chromatographed over silica gel, eluting with CH₂Cl₂/EtOH/NH₄OH 45/45/10, thereby yielding 9.2 g (58.7%) of a white solid.
¹H-NMR: δ (200MHz, CDCl₃): 8.58 (s.a., 1H); 7.27-7.19 (s.c., 2H); 6.99 (s.a., 1H) 6.96-6.85 (s.c., 1H) 2.95-2.88 (m, 4H); 2.71 (t, 2H); 2.48-2.36 (s.c., 6H) 2.10 (s.a., 1H); 1.96-1.80 (m, 2H)

### Example 40

### C-{3-[3-(3-amino-pyrrolidin-1-yl)-propyl]-1H-indol-5-Yl}-N-methyl-methanesulfonamide

To 8.7 g (0.02 mol) of 3-[5-[[(methylamino)sulfonyl]-methyl]-1H-indol-3-yl]propyl-p-toluenesulfonate prepared as described in Example 36, in 40 ml of EtOH was added a solution of 3-aminopyrrolidine prepared by the addition of 15.9 g (0.1 mol) of 3-aminopyrrolidine dihydrochloride over 50 ml of 2N NaOH. The reaction was held under reflux for 6 hours. It was concentrated in the rotary evaporator until the volume was reduced to 40 ml. 20 ml of 2N NaOH were added and the mixture extracted with 3 x 100 ml of CH₂Cl₂. The organic phase was dried and concentrated. The residue was chromatographed over silica gel, eluting with CH₂Cl₂/EtOH/NH₄OH 70/25/5, thereby yielding 3.8 g (54.2%) of a solid.
¹H-NMR: δ (200MHz; DMSO-d6): 10.8 (s.a., 1H); 7.52 (s, 1H); 7.30 (d, 1H); 7.12 (s.a., 1H) 7.04 (d, 1H); 4.37 (s, 2H); 3.36-3.26 (s.c., 1H); 2.72-2.36 (s.c., 9H) 2.54 (s, 3H); 2.16-2.06 (s.c., 1H); 2.02-1.90 (s.c., 1H) 1.82-1.70 (s.c., 2H); 1.40-1.24 (s.c., 1H)

### FORMULA X PRODUCTS

### Example 41: General Process

### 3-chloro-1-(1H-indol-3-yl)-propan-1-one

The general process described by Marcheses G.P. and F. Sacrini in Chim. Ind. (Milan) 51 (3), 258-262 (1969) was followed.

In a 500 ml round bottom flask there are placed 6.075 g (0.25 at-gr) of magnesium and 80 ml of dry ethyl ether. A crystal of I₂ and afterwards dropwise over 1 hour 19.25 ml (0.25 mol) of ethyl bromide were added. The resulting solution was cooled to -10°C and 29.4 g (0.2 mol) of indole dissolved in 100 ml of ethyl ether were added dropwise. At the end of the addition, 2 phases were formed. 100 ml of CH₂Cl₂ were added. The mixture was cooled to -20°C and were added slowly 17.8 g (0.14 mol) of 3-chloropropionyl chloride dissolved in 70 ml of ethyl ether. The mixture was stirred for 2 hours at 20°C. 150 ml of distilled water were added and the precipitated solid was filtered. 10.7 g of pure product were thus obtained.

The filter liquors were decanted and the organic phase was dried and concentrated. The residue was chromatographed over silica gel, eluting with AcOEt and a further 7.7 g of product were yielded.
¹H-NMR: δ (200MHz, DMSO-d6): 12.05 (s.a., 1H) 8.41 (d, 1H); 8.24-8.16 (s.c., 1H); 7.52-7.44 (s.c., 1H) 7.25-7.15 (s.c., 2H); 3.96 (t, 2H); 3.38 (t, 2H)

### Example 42

### 3-chloro-1-[1-(3-chloro-propionyl)-1H-indol-3-yl]-propan-1-one

When in the above reaction, instead of adding 0.14 mol of 3-chloropropionyl chloride, 0.2 mol of this product were added when adding the 150 ml of distilled water, 3.2 g of the product described in the previous Example precipitated out. The filter liquors were decanted and the organic phase was dried and concentrated. The residue was chromatographed over silica gel and eluting with heptane/AcOEt 2/1, 11.8 g of a solid were yielded.
¹H-NMR: δ (200MHz, DMSO): 8.97 (s, 1H); 8.42-8.36 (s.c., 1H); 8.26-8.22 (s.c., 1H); 7.46-7.38 (s.c., 2H) 4.02 (t, 2H); 3.99 (t, 2H); 3.76 (t, 2H) 3.52 (t, 2H)

### Example 43

### 3-chloro-1-[1-(3-chloro-propionyl)-5-fluoro-1H-indol-3-yl]-propan-1-one

Following the general process described above and starting out from 0.1 ml of 5-fluoroindole and 0.1 mol of 3-chloropropionyl chloride, subsequent purification in a column of silica gel, eluting with heptane/AcOEt 1/1 yielded 12.3 g (43.8%) of a solid.
¹H-NMR: (200MHz, DMSO.-d6): 9.08 (s, 1H); 8.38 (d,d, 1H); 8.18 (d,d, 1H) 7.17 (ddd, 1H); 4.08 (t, 2H) 3.96 (t, 2H), 3.49 (t, 2H) 3.37 (t, 2H)

### Example 44

### 3-chloro-1-(5-fluoro-1H-indol-4-yl)-propan-1-one

As by-product of the foregoing reaction anterior, 2.3 g (10.2%) of a solid were yielded on eluting the silica gel column with CH₂Cl₂/EtOH 9/1.
¹H-NMR: δ (200MHz, DMSO-d6): 12.14 (s.a., 1H) 8.44 (d, 1H); 7.86 (dd, 1H); 7.50 (dd, 1H) 7.10 (ddd, 1H); 3.98 (t, 2H); 3.36 (t, 2H).

### Example 45

### 3-chloro-1-[1-(3-chloro-propionyl)-5-methoxy-1H-indol-3-yl]propan-1-one

Following the general process described above and starting out from 0.1 ml of 5-methoxyindole and 0.1 ml of 3-chloropropionyl chloride, 15.7 g (47.8%) of a solid are yielded.
¹H-NMR: δ (200 MHz, CDCl₃): 8.25 (d, 1N) 8.01 (s, 1H); 7.82 (d, 1H); 7.02 (dd, 1H); 3.99 (t, 2H); 3.95 (t, 2H) 3.88 (s, 3H); 3.43 (t, 2H); 3.32 (t, 2H)

### Example 46

### 3-chloro-1-(5-methoxy-1H-indol-3-yl)-propan-1-one

As by-product of the foregoing reaction, 1.6 g (6.73%) of a solid were yielded on eluting the silica gel column with CH₂Cl₂/EtOH 9/1.
¹H-NMR: δ (200MHz, DMSO): 11.9 (s.a.,1H); 8.29 (d, 1H); 7.72 (d, 1H); 7.36 (d, 1H) 6.84 (dd, 1H); 3.94 (t, 2H) 3.76 (s, 3H); 3.35 (t, 2H); 3.84 (s, 3H)

### Example 47

### 3-[4-(5-methoxy-pyrimidin-4-yl)-piperazin-1-yl]-1-(1H-indol-3-yl)-propan-1-one hydrochloride

### Method A

3.1 g (0.015 ml) of 3-chloro-1-(1H-indol-3-yl)-propan-1-one and 2.9 g (0.015 ml) of 5-methoxy-4-piperazin-1-yl-pyrimidine were heated under reflux in 50 ml of ethanol for 24 hours. The mixture was cooled to room temperature and the precipitated white solid was filtered out, yielding 4.8 g (79.6%).
1H-NMR: δ (200 MHz, DMSO-d6): 12.06 (s.a., 1H); 8.43 (d, 1H); 8.29 (s, 1H); 8.22-8.16 (s.c., 1H); 8.09 (S, 1H); 7.52-7.46 (s.c., 1H) 7.26-7.14 (s.c., 2H); 3.85 (s, 3H); 3.85-3.75 (s.a., 2H); 3.60-3.30 (s.a., 4H); 3.30-3.15 (s.a., 2H) 3.1-2.9 (s.a., 2H); 2.95-2.75 (s.a., 2H).

### Method B

4.28 g of 3-chloro-1-[1-(3-chloropropionyl)-1H-indol-3-yl]-propan-1-one and 4 g of 5-methoxy-4-piperazin-1-yl-pyrimidine were heated under reflux in 50 ml of ethanol for 2 hours. The mixture was concentrated to dryness and the residue was chromatographed over silica gel, eluting with CH₂Cl₂/EtOH 8/2 to yield 3.7 g (56.5%) of product.

### Example 48

### 3-[4-(5-methoxy-pyrimidin-4-yl)-piperazin-1-yl]-1-(5-fluoro-1H-indol-3-yl)-propan-1-one hydrochloride

3.6 g of 3-chloro-1-[1-(3-chloropropionyl)-5-fluoro-1H-indol-3-yl]-propan-1-one (0.01 mol) and 4.9 g (0.025 mol) of 5-methoxy-4-piperazin-1-yl-pyrimidine were stirred in methanol under reflux for 3 hours. The mixture was concentrated to dryness and was divided between CH₂Cl₂ and water. The precipitate formed was filtered and was washed first with CH₂Cl₂ and afterwards with cold water. 4.2 g (93%) of a white solid were yielded.
¹H-NMR: δ (200 MHz, DMSO): 12.42 (s.a., 1H); 8.71 (s, 1H); 8.50 (d, 1H); 8.25 (s, 1H) 7.83 (dd, 1H); 7.53 (dd, 1H) 7.10 (ddd; 1H); 5.00 (s.a., 1H); 3.92 (s, 3H) 4.30-2.80 (s.a., 8H)

### Example 49

### 3-[4-(5,6-dimethoxy-pyrimidin-4-yl)-piperazin-1-yl]-1-(5-fluoro-1H-indol-3-yl)-propan-1-one hydrochloride

2.8 g (0.01 ml) of 3-chloro-1-[1-(3-chloro-propionyl)-5-fluoro-1H-indol-3-yl]-propan-1-one and 6.8 g (0.03 mol) of 5,6-dimethoxy-4-piperazin-1-yl-pyrimidine were stirred in 50 ml of THF for 12 hours at room temperature. The precipitated solid was filtered out, thereby yielding 3.1 g. The filter liquors were concentrated to dryness and were chromatographed over silica gel, eluting with CH₂Cl₂/EtOH 95/5 thereby yielding a further 0.9 g of product.
¹H-NMR: δ (200MHz, DMSO-d6): 12.08 (s.a., 1H); 8.44 (d, 1H); 8.07 (s, 1H); 7.87 (dd, 1H); 7.49 (dd, 1H); 7.06 (ddd, 1H) 3.90 (s, 3H); 3.80-3.60 (s.a., 4H); 3.62 (s., 3H); 3.12 (t, 2H); 2.82 (t, 2H); 2.78-2.58 (s.a., 4H).

### Example 50

### 3-[4-(5,6-dimethoxy-pyrimidin-4-yl)-piperazin-1-yl]-1-(5-methoxy-1H-indol-3-yl)-propan-1-one

### Method A

1.19 g (5 mmol) of 3-chloro-1-(5-methoxy-1H-indol-3-yl)-propan-1-one and 1.12 g (5 mmol) of 5,6-dimethoxy-4-piperazin-1-yl-pyrimidine and 0.53 g (5 mmol) of sodium carbonate were stirred under reflux in 50 ml of THF and 10 ml of water for 24 hours. The reaction mixture was concentrated to dryness and 50 ml of distilled water were added, followed by extraction with 3 x 50 ml of dichloromethane. The extracts were dried with sodium sulfate, were filtered and concentrated. The residue was chromatographed over silica gel, eluting with CH₂Cl₂/EtOH 95/5, to yield 1.85 g (87%) of a white solid.
¹H-NMR: δ (200MHz, DMSO): 11.92 (s.a., 1H); 8.36 (d, 1H); 8.04 (s, 1H); 7.72 (d, 1H); 7.37 (d, 1H); 6.82 (dd, 1H) 3.89 (s, 3H); 3.78 (s, 3H); 3.70-3.60 (s.c., 4H); 3.60 (s, 3H) 3.01 (t, 2H); 2.70 (t, 2H); 2.56-2.44 (s.c., 4H)

### Method B

Starting out from 1.64 g (5 mmol) of 3-chloro-1-[1-(3-chloropropionyl)-5-fluoro-1H-indol-3-yl]-propan-1-one, 3.36 g (15 mmol) of 5,6-dimethoxy-4-(piperazin-1-yl)-pyrimidine and 1.6 g (15 mmol) of sodium carbonate and proceeding as in Method A, 1.74 g (81.8%) of the same product were yielded.

### FORMULA I PRODUCTS

### Method A: General Process

### Example 51:

### C-(3-{3-[4-(5,6-dimethoxy-pyrimidin-4-yl)-piperazin-1-yl]-propyl}-1H-indol-5-yl)-N-methyl-methanesulfonamide

To 2.18 g (5 mmol) of 3-[5-[[(methylamino)sulfonyl]methyl]-1H-indol-3-yl]propyl p-toluenesulfonate in 50 ml of anhydrous acetonitrile, were added 1.12 g (5 mmol) of 5,6-dimethoxy-4-piperazin-1-yl-pyrimidine and 0.5 g (6 mmol) of sodium bicarbonate. The reaction was held under reflux for 5 hours. The precipitated organic salts were filtered. The filter liquors were concentrated to dryness, 50 ml of 2N NaOH were added, followed by extraction with 3 x 50 ml of CH₂Cl₂. The organic phase was dried with sodium sulfate, was filtered and concentrated. The residue was chromatographed over silica gel, eluting with CH₂Cl₂/EtOH 9/1 to yield 2.01 g (86%) of a solid. m.p.:156-157°C
IR(KBr): 3000; 1570 cm⁻¹
¹H-NMR: δ (200MHz, CDCl₃): 8.3 (s.a., 1H); 8.10 (s, 1H); 7.61 (s, 1H); 7.35 (d, 1H) 7.18 (d, 1H); 7.02 (s, 1H); 4.35 (s, 2H); 4.22 (q, 1H); 3.97 (s, 3H); 3.85-3.75 (s.c., 4H); 3.69 (s, 3H); 2.79 (t, 2H); 2.61 (d, 3H); 2.62-2.52 (s.c., 4H) 2.51 (t, 2H); 2.05-1.90 (s.c., 2H).

### Example 52

### C-(3-{3-[4-(2-chloro-pyrimidin-4-yl)-piperazin-1-yl]-propyl}-1H-indol-5-yl)-N-methylmethanesulfonamide

Prepared in a similar manner by replacing the 5,6-dimethoxy-4-piperazin-1-yl-pyrimidine with the equivalent amount of 2-chloro-4-piperazin-1-yl-pyrimidine.
m.p: 67-68°C
IR(KBr): 1590 cm⁻¹
¹H-NMR: δ (200MHz, DMSO-d6): 10.82 (s.a., 1H); 8.07 (d, 1H); 7.54 (s, 1H); 7.34 (d, 1H); 7.16 (s, 1H); 7.08 (d, 1H); 6.85 (d, 1H) 6.81 (q, 1H); 4.38 (s, 2H); 3.70-3.56 (s.a., 4H); 2.70 (t, 2H); 2.56 (d, 3H); 2.50-2.30 (s.a., 6H); 1.96-1.80 (s.c., 2H)

### Example 53

### C-(3-{3-[4-(6-chloro-5-methoxy-pyrimidin-4-yl)-2-methyl-piperazin-1-yl]-propyl}-1H-indol-5-yl)-N-methyl-methanesulfonamide

Prepared by the general process described in Example 51 by replacing the 5,6-dimethyl-4-piperazin-1-yl-pyrimidine with the equivalent amount of 4-chloro-5-methoxy-6-(3-methyl-piperazin-1-yl)-pyrimidine.
m.p.: 60-61°C
IR(KBr): 1560 cm⁻¹
¹H-NMR: δ (200 MHz, DMSO-d6): 10.82 (s, 1H); 8.14 (s, 1H); 7.53 (s, 1H); 7.34 (d, 1H); 7.16 (s, 1H); 7.06 (d, 1H); 6.79 (q, 1H); 4.38 (s, 2H); 4.16 (t, 2H); 3.84 (s, 3H); 3.46-3.38 (s.c., 1H); 3.16-3.04 (s.c., 1H); 2.94-2.80 (s.c., 1H); 2.80-2.60 (s.c., 3H); 2.56 (d, 3H)2.56-2.40 (s.c.,1H); 2.38-2.20 (s.a., 2H); 1.86-1.76 (s.c., 2H)

### Example 54

### C-(3-{3-[1-(6-chloro-5-methoxy-pyrimidin-4-yl)-pyrrolidin-3-ylamino]-propyl}-1H-indol-5-yl)-N-methyl-methanesulfonamide

Prepared by replacing the 5,6-dimethoxy-4-piperazin-1-yl-pyrimidine with 1-(6-chloro-5-methoxy-pyrimidin-4-yl)-pyrrolidin-3-yl-amine in the general process described in Example 51.
m.p: 57-58°C
IR(KBr): 1560 cm⁻¹
1H-NMR: δ (200MHz, DMSO-d6): 10.82 (s, 1H); 8.06 (s, 1H); 7.52 (s, 1H); 7.32 (d, 1H); 7.12 (s, 1H); 7.08 (d, 1H); 6.80 (q, 1H); 4.38 (s, 2H); 3.80-3.60 (s.c., 2H); 3.66 (s, 3H); 3.50-3.20 (s.c., 3H) 3.30 (s.a., 1H); 2.74 (t, 2H); 2.60-2.50 (m, 2H) 2.50 (d, 3H); 2.10-1.90 (s.c., 1H); 1.90-1.70 (s.c., 3H)

By replacing the 5,6-dimethoxy-4-(1-piperazinyl)pyrimidine with the corresponding amine and proceeding as described in Example 51, the following products are yielded:

### Example 55

### C-(3-{3-[1-(5-methoxy-pyrimidin-4-yl)-pyrrolidin-3-ylamino]-propyl}-1H-indol-5-yl)-N-methyl-methanesulfonamide

m.p.: 81-82°C
IR(KBr): 1580 cm⁻¹
¹H-NMR: δ (200 MHz, DMSO-d6): 10.80 (s.a, 1H); 8.10 (s, 1H); 7.82 (s, 1H); 7.50 (s, 1H) 7.32 (d, 1H); 7.12 (s, 1H); 7.08 2(d, 1H); 6.77 (q, 1H); 4.34 (s, 2H); 3.81-3.58 (s.c., 3H); 3.74 (s, 3H); 3.50-3.38 (s.c., 1H); 3.30-3.20 (s.c., 1H) 3.30 (s.a., 1H); 2.70 (t, 2H); 2.64-2.50 (s.c., 2H); 2.46 (d, 3H); 2.04-1.84 (s.c., 1H) 1.84-1.60 (s.c., 3H)

### Example 56

### C-(3-{3-[1-(5,6-dimethoxy-pyrimidin-4-yl)-pyrrolidin-3-ylamino]-propyl}-1H-indol-5-yl)-N-methyl-methanesulfonamide

m.p.: 64-65°C
IR(KBr): 1580 cm⁻¹
¹H-NMR: δ (200MHz, DMSO-d6): 10.82 (s.a., 1H); 7.98 (s, 1H); 7.54 (s, 1H); 7.32 (d, 1H); 7.12 (s, 1H); 7.07 (d, 1H); 6.81 (s.a., 1H); 4.34 (s, 2H); 3.86 (s, 3H); 3.80-3.50 (s.c., 3H); 3.61 (s, 3H); 3.42-3.22 (s.c., 2H); 2.72 (t, 2H); 2.64-2.50 (s.c., 2H); 2.46 (s.a., 3H); 2.06-1.90 (s.c., 1H) 1.86-1.60 (s.c., 3H)

### Example 57

### C-(3-{3-[3-(5-methoxy-pyrimidin-4-ylamino-pyrrolidin-1-yl]-propyl}-1H-indol-5-yl)-N-methyl-methanesulfonamide

m.p.: 62-63°C
IR(KBr): 1600 cm⁻¹
¹H-NMR: δ (200 MHz, DMSO-d6): 10.81 (s.a., 1H); 8.10 (s, 1H); 7.79 (s, 1H); 7.49 (s, 1H); 7.31 (d, 1H); 7.12 (s.a., 1H); 7.08 (d, 1H); 6.79 (q, 1H); 6.74 (d, 1H); 4.50-4.40 (s.c., 1H); 4.32 (s, 2H); 3.80 (s, 3H); 2.82-2.58 (s.c., 4H); 2.50-2.34 (s.c., 4H); 2.46 (d, 3H); 2.10-2.04 (s.c., 1H); 1.84-1.64 (s.c., 3H)

### Example 58

### C-(3-{3-[3-(5,6-dimethoxy-pyrimidin-4-ylamino)-pyrrolidin-1-yl]propyl}-1H-indol-5-yl)-N-methyl-methanesulfonamide

m.p.: 72-73°C
IR(KBr): 1600 cm⁻¹
¹H-NMR: δ (200 MHz, DMSO-d6): 10.82 (s.a., 1H); 7.98 (s, 1H); 7.51 (s, 1H); 7.32 (d, 1H); 7.12 (s.a., 1H); 7.08 (d, 1H); 6.80 (q, 1H); 6.69 (d, 1H); 4.52-4.35 (s.c., 1H) 4.31 (s, 2H); 3.85 (s, 3H); 3.62 (s, 3H); 2.90-2.75 (s.c., 1H); 2.75-2.60 (s.c., 3H); 2.50 (d, 3H); 2.50-2.35 (s.c., 4H);2.20-2.05 (s.c., 1H); 1.90-1.65 (s.c., 3H).

### Example 59

### C-[3-(3-{2-[5-methoxy-pyrimidin-4-yl)-methylaminolethylamino}-propyl)-1H-indol-5-yl]-N-methyl-methanesulfonamide.

m.p: 55-56°C
IR(KBr): 1600 cm⁻¹
¹H-NMR: δ (200MHz, DMSO-d6): 10.90 (s.a., 1H); 8.14 (s, 1H), 7.80 (s, 1H); 7.54 (s, 1H); 7.39 (d, 1H); 7.12 (s, 1H); 7.11 (d, 1H); 6.88 (s.a., 1H); 6.78 (t, 1H); 4.40 (s, 2H); 3.84 (s, 3H); 3.54-3.36 (s.c., 2H); 2.72 (t, 2H); 2.57 (s.a., 5H); 2.43 (t, 2H); 2.24 (s,3H); 1.90-1.76 (s.c., 2H).

### Example 60

### C-[3-(3-{2-[(5,6-dimethoxy-pyrimidin-4-yl)-methylamino]-ethylamino}-propyl)-1H-indol-5-yl]-N-methyl-methanesulfonamide

m.p: 50-51°C
IR(KBr): 1580 cm⁻¹
¹H-NMR: δ (200MHz, DMSO-d6): 10.82 (s.a., 1H); 8.00 (s, 1H); 7.56 (s, 1H); 7.34 (d, 1H); 7.12 (s, 1H); 7.11 (d, 1H) 6.80 (s.a., 1H); 4.38 (s, 2H); 3.85 (s, 3H); 3.70-3.60 (s.c., 2H); 3.62 (s, 3H); 3.24 (s.a., 1H); 3.17 (s, 3H); 2.74 (t, 2H); 2.66-2.46 (s.c., 4H); 2.54 (s, 3H); 1.84-1.70 (s.c., 2H)

### Example 61

### C-{3-[3-({2-[(5-methoxy-pyrimidin-4-yl)-methyl-amino]-ethyl]-methyl-amino)-propyl]-1H-indol-5-yl}-N-methyl-methanesulfonamide

m.p: 54-55°C
IR(KBr): 1580 cm⁻¹
¹H-NMR: δ (200MHz, DMSO-d6): 10.81 (s.a., 1H); 8.14 (s, 1H); 7.84 (s, 1H); 7.46 (s., 1H) 7.30 (d, 1H); 7.09 (s, 1H); 7.08 (d, 1H); 6.80 (q, 1H); 4.35 (s, 2H); 3.77 (s, 3H); 3.70 (t, 2H); 3.09 (s, 3H); 2.62 (t, 2H); 2.56-2.42 (s.c., 5H); 2.38 (t, 2H); 2.19 (s, 3H); 1.80-1.62 (s.c., 2H)

### Example 62

### C-{3-[3-({2-[(5,6-dimethoxy-pyrimidin-4-yl)-methyl-amino]-ethyl}-methyl-amino)-propyl]-1H-indol-5-yl}-N-methyl-methanesulfonamide

m.p: 47-48°C
IR(KBr): 1580 cm⁻¹
¹H-NMR: δ (200MHz, DMSO-d6): 10.8 (s,a, 1H); 7.99 (s, 1H); 7.48 (s, 1H); 7.32 (d, 1H); 7.07 (s, 1H); 7.06 (d, 1H); 6.80 (q, 1H); 4.34 (s, 2H); 3.85 (s, 3H); 3.64 (t, 2H); 3.60 (s, 3H), 3.12 (s, 3H); 2.62 (t, 2H); 2.56-2.44 (s.c., 5H); 2.39 (t, 2H); 2.20 (s, 3H); 1.82-1.64 (s.c., 2H)

### Example 63

### C-[3-(3-{2-[(5,6-dimethoxy-pyrimidin-4-yl)-amino]ethylamino}-propyl)-1H-indol-5-yl]-N-methyl-methanesulfonamide

m.p.: 56-57°C
IR(KBr): 1600 cm⁻¹
¹H-NMR: δ (200MHz, DMSO-d6): 10.8 (s.a., 1H); 7.98 (s, 1H); 7.50 (s, 1H); 7.32 (d, 1H); 7.11 (s, 1H); 7.09 (d, 1H); 6.80 (s.a., 1H); 4.36 (s, 2H); 4.84 (s, 3H); 4.62 (s, 3H); 4.41 (t, 2H); 4.40-4.25 (s.c., 2H); 2.76-2.46 (s.c., 9H); 1.84-1.78 (s.c., 2H).

### Example 64

### C-(3-{3-[4-(6-methylamino-5-methoxy-pyrimidin-4-yl)-piperazin-1-yl]-propyl}-1H-indol-5-yl)-N-methyl-methanesulfonamide

m.p: 86-87°C
IR(KBr): 1600 cm⁻¹
¹H-NMR: δ (200MHz, DMSO-d6); 10.81 (s.a., 1H); 7.86 (s, 1H); 7.50 (s, 1H); 7.30 (d, 1H); 7.14 (s, 1H); 7.09 (d, 1H); 6.80 (q, 1H); 6.61 (q, 1H); 4.34 (s, 2H); 3.54 (s, 3H); 3.54-3.52 (s.c., 4H); 2.79 (d, 3H); 2.70 (t, 2H); 2.54 (d, 3H); 2.50-2.40 (s.c., 4H) 2.37 (t, 2H) 1.90-1.76 (s.c., 2H).

### Example 65

### 5-fluoro-3-{3-[4-(6-chloro-5-methoxy-pyrimidin-4-yl)-2-methyl-piperazin-1-yl]-propyl}-1H-indole

m.p: 49-50°C
IR(KBr): 1570 cm⁻¹
¹H-NMR: δ (200MHz, CDCl₃): 8.35 (s.a., 1H); 8.17 (s, 1H); 7.30-7.20 (s.c., 2H); 7.03 (s.a., 1H) 6.95-6.85 (s.c., 1H); 4.40-4.20 (s.c., 2H); 3.71 (s, 3H); 3.48-3.35 (s.c., 1H); 3.15-3.05 (s.c., 1H); 2.97-3.65 (s.c., 4H); 2.60-2.30 (s.c., 3H); 1.95-1.80 (s.c., 2H); 1.06 (d,3H)

### Example 66

### 5-fluoro-3-{3-[4-(2-chloro-pyrimidin-4-yl)-piperazin-1-yl]-propyl}-1H-indole

m.p: 63-64°C
IR(KBr): 1580 cm⁻¹
¹H-NMR: δ (200MHz, CDCl₃): 8.06 (s.a., 1H); 8.03 (d, 1H): 7.31-7.22 (s.c., 2H); 7.05 (s.a., 1H); 6.98-6.88 (s.c., 1H); 6.39 (d, 1H); 3.72-3.62 (s.a., 4H); 2.77 (t, 2H); 2.53-2.45 (s.c., 4H); 2.44 (t, 2H); 1.99-1.84 (m, 2H)

### Example 67

### 3-{3-[4-(6-chloro-5-methoxy-pyrimidin-4-yl)-piperazin-1-yl]-propyl}-5-fluoro-1H-indole hydrochloride

5.2 g (0.02 ml) of 5-fluoro-3-(3-piperazin-1-yl-propyl)-1H-indole prepared as described in Example 39 and 3.6 g (0.02 mol) of 4,6-dichoro-5-methoxypyrimidine were heated under reflux in 50 ml of acetonitrile for 24 hours. The mixture was allowed to reach room temperature and the precipitated solid was filtered and was washed with 20 ml of acetonitrile. 4.3 g of product were thereby yielded. The filter liquors were concentrated to dryness and the residue was chromatographed over silica gel. A further 1.4 g of product were recovered by eluting with CH₂Cl₂/MeOH 9/1. Total 5.7 g (Yield. 64.7%)
m.p: 216-217°C
IR(KBr): 1560; 2450; 2550 cm⁻¹
¹H-NMR: δ (200MHz, DMSO): 11.02 (s.a., 1H); 8.24 (s, 1H); 7.38-7.24 (s.c., 3H); 6.96-6.84 (s.c., 1H): 4.65-4.40 (s.a., 1H); 3.70 (s, 3H); 3.44-3.22 (s.a., 8H); 3.16-3.04 (s.a., 2H); 2.78-2.64 (s.a., 2H); 2.10-2.00 (s.a., 2H)

By following the same process and replacing the 4,6-dichloro-5-methoxy-pyrimidine with the corresponding substituted chloro-pyrimidine, the following products were prepared with yields ranging from 65-82%.

### Example 68

### 3-{3-[4-(2-chloro-5-methyl-pyrimidin-4-yl)-piperazin-1-yl]-propyl}-5-fluoro-1H-indole hydrochloride

m.p.: 213-214
IR(KBr): 1580; 2450; 2530 cm⁻¹
¹H-NMR: δ (200MHz, DMSO): 11.02 (s.a., 1H); 8.14 (s,1H); 7.40-7.26 (s.c., 3H); 6.98-6.84 (s.c., 1H); 4.20-4.00 (s.a., 1H); 3.65-3.40 (s.a., 4H); 3.20-3.00 (s.a., 6H); 2.78-2.66 (s.a., 2H); 2.20 (s, 3H); 2.16-2.00 (s.a., 2H).

### Example 69

### 6-chloro-4-{4-(5-fluoro-1H-indol-3-yl)-propyl]-piperazin-1-yl}-pyrimidin-5-ylamine hydrochloride

m.p.: 100-101°C
IR(KBr): 1550; 2450 cm⁻¹
¹H-NMR:(200MHz, DMSO): 11.00 (s.a., 1H); 8.00 (s, 1H); 7.36-7.24 (s.c., 3H): 6.96-6.86 (s.c., 1H) 5.19 (s.a., 2H); 3.56-3.24 (s.a., 4H); 3.10-2.60 (s.a., 6H); 2.70 (t, 2H), 2.04-1.94 (s.a., 2H)

### Example 70

### 5-fluoro-3-{3-[4-(2-methyl-5-methoxy-pyrimidin-4-yl)-piperazin-1-yl]-propyl}-1H-indole

m.p: 54-55°C
IR(KBr): 1590, 2440, 2560 cm⁻¹
¹H-NMR:(200MHz, DMSO): 11.04 (s.a., 1H); 8.02 (s, 1H); 7.38-7.26 (s.c., 3H); 6.94-6.84 (s.c., 1H); 4.60-4.30 (s.a., 1H); 3.82 (s, 3H); 3.80-3.00 (s.a., 10H); 2.76-2.64 (s.a., 2H); 2.39 (s, 3H); 2.20-2.00 (s.a., 2H)

### Example 71

### 3-{3-[4-(6-chloro-pyrimidin-4-yl)-piperazin-1-yl]-propyl}-5-fluoro-1H-indole hydrochloride

m.p: 228-229°C
IR(KBr): 1590, 2490, 2600 cm⁻¹
¹H-NMR: 11.02 (s.a., 1H); 8.42 (s, 1H), 7.40-7.26 (s.c., 3H); 7.12 (s, 1H); 6.96-6.84 (s.c., 1H); 4.60-4.40 (s.a., 1H); 3.65-3.25 (s.a., 6H); 3.18-2.90 (s.a., 4H); 2.78-2.62 (s.a., 2H); 2.20-2.00 (s.a., 2H)

### Example 72

### C-(3-{3-[3-(6-chloro-5-methoxy-pyrimidin-4-ylamino)-pyrrolidin-1-yl]-propyl}-1H-indol-5-yl)-N-methyl-methanesulfonamide

2.1 g (6 mmol) of C-{3-[3-(3-amino-pyrrolidin-1-yl)-propyl]-1H-indol-5-yl}-N-methyl-methanesulfonamide prepared as described in Example 40 and 1.07 g (6 mmol) of 4,6-dichloro-5-methoxypyrimidine were stirred in 50 ml of ethanol and 10 ml of water a 40°C for 3 hours and at room temperature overnight. The mixture was concentrated to dryness. The residue was treated with 50 ml of 2N NaOH and was extracted with 3 x 50 ml of dichloromethane. The organic phase was dried with sodium sulfate, was filtered and concentrated. The residue was chromatographed over silica gel, eluting with CH₂Cl₂/EtOH/NH₂OH 9/1/1 and yielding 2 g (68%) of product.
m.p: 66-67°C
IR(KBr): 1580 cm⁻¹
¹H-NMR:(200MHz,DMSO): 10.82 (s.a., 1H); 8.06 (s, 1H); 7.54 (s.a., 1H); 7.50 (s, 1H); 7.32 (d, 1H); 7.14 (s.a., 1H); 7.08 (d, 1H); 6.99 (q, 1H); 4.56-4.38 (s.c., 1H) 7.30 (s, 2H); 3.69 (s, 3H); 2.84-2.30 (s.c., 10H); 2.22-2.06 (s.c., 1H); 1.94-1.64 (s.c., 3H); 1.30-1.20 (s.c., 1H)

### Example 73

### 3-{3-[4-(5,6-dimethoxy-pyrimidin-4-yl)-piperazin-1-yl]-propyl}-5-fluoro-1H-indole

4.4 g (10 mmol) of the product prepared in Example 67 were added over a solution of 1.35 g (25 mmol) of sodium methoxide in 50 ml of absolute methanol. The reaction was held under reflux for 8 hours. It was concentrated to dryness, the residue was treated with 50 ml of water and was extracted with 3 x 50 ml of CH₂Cl₂. The extracts were dried, concentrated and were chromatographed over silica gel, eluting with CH₂Cl₂/MeOH 95/5 and yielding 2.48 g (62%) of a white solid.
m.p.: 155-156°C
IR(KBr): 1580 cm⁻¹
¹H-NMR: (200MHz, CDCl₃): 8.20 (s.a., 1H); 8.10 (s, 1H); 7.30-7.21 (s.c., 2H); 7.03 (s.a., 1H); 6.96-6.86 (s.c., 1H); 3.99 (s, 3H); 3.81-3.75 (s.c., 4H); 3.69 (s, 3H); 2.75 (t, 2H); 2.58-2.48 (s.c., 4H); 2.45 (t, 2H); 1.99-1.84 (m, 2H)

### Example 74

### 5-fluoro-3-{3-[4-(5-methyl-2-methoxy-pyrimidin-4-yl)-piperazin-1-yl]-propyl}-1H-indole dihydrochloride

Following the process described in the previous Example and starting out from the product prepared in Example 68 (5 mmol) a product was obtained which was dissolved in methanol and to which were added 2 equivalents of hydrochloric acid. In this manner. 1.8 g (79%) of the expected product were yielded in dihydrochloride form.
m.p.: 186-187°C
IR(KBr): 1610; 2550 cm⁻¹
¹H-NMR: (200MHz, D₂O): 7.85 (s, 1H); 7.98-7.82 (s.c., 2H); 7.27 (s, 1H); 7.06-6.96 (s.c., 1H); 4.01 (s, 3H); 3.70-3.15 (s.a., 10H); 2.85 (t, CH₂); 2.22 (s, 3H); 2.20-2.08 (s.c., 2H)

### Example 75

### 4-{4-[3-(5-fluoro-1H-indol-3-yl)-propyl]-piperazin-1-yl}-6-methoxy-pyrimidin-5-yl-amine dihydrochloride

The product was synthesized starting from the product prepared in Example 69 and proceeding as described in the previous Example.
m.p.: 156-157°C
IR(KBr): 1620; 2560; 2650 cm⁻¹
¹H-NMR:(200MHz, D₂O): 8.08 (s, 1H); 7.48-7.32 (s.c., 2H); 7.28 (s, 1H); 7.05-6.95 (s.c., 1H); 4.06 (s, 3H); 3.85-3.55 (s.c., 4H); 3.32-3.20 (s.c., 6H); 2.85 (t, 2H); 2.25-2.08 (s.c., 2H)

### Example 76

### 3-{3-[4-(5,6-dimethoxy-pyrimidin-4-yl)-2-methyl-piperazin-1-yl]-propyl}-5-fluoro-1H-indole

Starting out from 4.17 g (0.01 ml) of the product prepared in Example 65 and following the process described in Example 73, 3.9 g (94.4%) of product were yielded.
m.p: 51-52°C
IR(KBr): 1580 cm⁻¹
¹H-NMR:(200MHz, CDCl₃): 8.37 (s.a., 1H); 8.10 (s, 1H); 7.27-7.19 (s.c., 2H); 7.02 (s.a., 1H) 6.97-6.87 (s.c., 1H); 4.30-4.10 (s.c., 2H) 3.99 (s, 3H); 3.70 (s, 3H); 3.42-3.26 (s.c., 1H) 3.10-2.30 (s.c., 8H); 1.96-1.82 (s.c., 2H). 1.07 (d, 3H)
The dihydrochloride of this product was prepared in a manner similar to that described in Example 74.

### Example 77

### 4-{4-[3-(5-fluoro-1H-indol-3-yl)-propyl]-piperazin-1-yl}-pyrimidin-5-yl-amine hydrochloride

To 2.5 g (5.88 mmol) of the product prepared in Example 69 in 50 ml of methanol, were added 0.3 g of 10% Pd/C paste. The reaction was placed in a hydrogenation apparatus at 60 atm. for 15 hours. It was filtered over decalite and the decalite was washed with 50 ml of methanol. The filter liquors were concentrated to dryness and the residue was chromatographed over silica gel, eluting with CH₂Cl₂/EtOH 9/1, yielding 0.9 g (39.2%) of product as hydrochloride.
m.p.: 219-220°C
IR(KBr): 1580; 2600 cm⁻¹
¹H-NMR:(200MHz, DMSO): 11.18 (s.a., 1H); 8.18 (s, 1H); 7.96 (s, 1H); 7.40-7.35 (s.c., 3H); 6.96-6.82 (s.c., 1H); 5.10 (s.a., 2H) 3.70-2.90 (s.c., 10H); 2.78-2.62 (s.a., 2H); 2.14-2.00 (s.a., 2H).

### Example 78

### C-(3-{3-[3-(5,6-dimethoxy-pyrimidin-4-yl)-piperazin-1-yl]-1-methyl-1H-indol-5-yl)-N-methyl-methanesulfonamide

To a solution of 4.9 g (10 mmol) of the product prepared in Example 51 in 100 ml of anhydrous N,N-dimethylformamide under a nitrogen flow, were added 0.8 g (20 mmol) of 60% sodium hydride in mineral oil. The mixture was cooled to-13°C and were added dropwise 0.47 ml (5 mmol) of dimethyl sulfate in 15 ml of N,N-dimethylformamide. Stirring was continued for 1 hour, allowing the temperature to rise to 15°C. It was cooled again to -5°C and were added a further 0.2 ml (2.1 mmol) of dimethyl sulfate in 15 ml of DMF. Stirring was continued for a further 1 hour. 15.4 ml of 25% ammonia were added and the resulting solution was concentrated in vacuo. To the residue were added 50 ml of water and it was extracted with 3 x 50 ml of dichloromethane. The organic phase was washed with 2 x 50 ml of 25% ammonia. The organic phase was dried and concentrated and the residue was chromatographed over silica gel.

Eluting with CH₂Cl₂/EtOH 95/5 yielded 0.7 g (13.9%)
m.p: 60-61°C
IR(KBr): 1590 cm⁻¹
¹H-NMR: (200 MHz, DMSO): 8.05 (s,1H); 7.52 (s.a., 1H); 7.37 (d, 1H); 7.14 (d, 1H); 7.13 (s, 1H); 6.79 (q, 1H). 4.36 (s, 2H); 3.88 (s, 3H); 3.74 (s, 3H); 3.72-3.62 (s.c., 4H); 3.60 (s, 3H); 2.69 (t, 2H) 2.50 (d, 3H); 2.48-2.40 (s.c., 4H); 2.35 (t, 2H); 1.90-1.75 (s.c., 2H).

### Example 79

### C-(3-{3-[3-(5,6-dimethoxy-pyrimidin-4-yl)piperazin-1-yl]-1-methyl-1H-indol-5-yl)-N,N-dimethyl-methanesulfonamide

The product is prepared from the above reaction by eluting the silica gel column with CH₂Cl₂/EtOH 98/2. 1.6 g of product were yielded in form of an oil which solidifies on standing.
m.p: 97-98°C
¹H-NMR:(200 MHz, DMSO): 8.06 (s, 1H); 7.58 (s, 1H); 7.37 (d, 1H); 7.15 (d, 1H); 7.11 (s, 1H); 4.43 (s, 2H); 3.89 (s, 3H) 3.72 (s, 3H); 3.72-3.62 (s.c., 4H) 3.60 (s, 3H); 2.68 (s, 6H); 2.52-2.42 (s.c., 6H); 2.36 (t, 2H); 1.88-1.76 (s.c., 2H).

### Example 80

### 3-{3-[4-(5,6-dimethoxy-pyrimidin-4-yl)-piperazin-1-yl]-propyl}-5-methoxy-1H-indole

To 2.1 g (5 mmol) of the product prepared in Example 50 (previously converted into a base by dilution in water basified with NaOH and extraction with CH₂Cl₂) in 60 ml of THF, were added 0.38 g (10 mmol) of lithium hydride and aluminium. The reaction was held under reflux for 4 hours. The excess HLA was hydrolyzed and the precipitated salts were filtered and were washed in 40 ml of THF. The filter liquors were concentrated to dryness. The residue was treated with 50 ml of water and extracted with 3 x 50 ml of CH₂Cl₂. The extracts were dried and concentrated. The residue was chromatographed over silica gel, eluting with CH₂Cl₂/EtOH 95/5 and yielding 0.9 g (43.7%) of a white solid.
m.p: 151-152°C
IR(KBr): 1590 cm⁻¹
¹H-NMR:(200MH-CDCl₃): 8.10 (s, 1H); 7.96 (s.a., 1H) 7.23 (d, 1H); 7.05 (d, 1H); 6.97 (s.a., 1H); 6.84 (dd, 1H); 3.99 (s, 3H); 3.87 (s, 3H); 3.80-3.72 (s.c., 4H) 3.70 (s, 3H); 2.77 (t, 2H); 2.56-2.48 (s.c., 4H), 2.49 (t, 2H); 2.00-1.88 (s.c., 2H)

### Example 81

### 1-(1H-indol-3-yl)-3-[4-(5-methoxy-pyrimidin-4-yl)-piperazin-1-yl]-propan-1-ol

36 g (7 mmol) of the product prepared in Example 47 were dissolved in 50 ml of methanol and were added 0.53 g (14 mmol) of sodium borohydride. The reaction was conducted under reflux and was held thus for 6 hours and then at room temperature overnight. It was concentrated to dryness, was treated with 100 ml of water and was extracted with 4 x 50 ml of dichloromethane. The extracts were dried and concentrated and the residue was chromatographed over silica gel, eluting with CH₂Cl₂/EtOH 9/1 and yielding 1.6 g (62.2%) of product
IR(KBr): 1590 cm⁻¹
¹H-NMR:(200MHz, DMSO): 10.85 (s.a., 1H); 8.24 (s, 1H); 8.04 (s, 1H); 7.63 (d, 1H); 7.37 (d, 1H); 7.20 (s, 1H); 7.10-6.92 (s.c., 2H); 5.05 (s.a., 1H); 4.90 (t, 1H); 3.82 (s, 3H); 3.70-3.60 (s.c., 4H); 2.46-2.30 (s.c., 6H); 2.02-1.90 (s.c., 2H)

### Example 82

### 3-{3-[4-(5-methoxy-pyrimidin-4-yl)-piperazin-1-yl]propenyl}-1H-indole

1.2 g (3.3 mmol) of the product prepared in the previous Example were heated a 80°C for 6 hours, with the water produced during the reaction being driven off in vacuo. The residue was chromatographed over silica gel, eluting with CH₂Cl₂/EtOH 95/5, yielding 0.4 g (34.7%) of a solid of m.p.: 64-65°C, which is a mixture of CIS and TRANS isomers.

### Example 83

### 5-fluoro-3-{3-[4-(5-methoxy-pyrimidin-4-yl)-piperazin-1-yl]-propenyl}-1H-indole

To 2.2 g (5.2 mmol) of the product prepared in Example 48 in form of free base dissolved in 50 ml of methanol were added 0.43 g (11.4 mmol) of sodium borohydride. The reaction was held under reflux for 6 hours and thereafter at room temperature overnight. It was concentrated to dryness and the residue was treated with 100 ml of water and extracted with 3 x 50 ml of dichloromethane. The extracts were dried and concentrated. The residue was chromatographed over silica gel, eluting with CH₂Cl₂/EtOH 93/7, yielding 0.8 g (41.9%), of an oil which is a mixture of CIS and TRANS isomers.
m.p.: 70-71°C

### Example 84

### 1-(5-fluoro-1H-indol-3-yl)-3-[4-(5-methoxy-pyrimidin-4-yl)piperazin-1-yl]-propan-1-ol

The product was prepared by following the process described in the previous Example, by eluting the silica gel column with CH₂Cl₂/EtOH/NH₄OH 9/1/1. 0.4 g of a solid of m.p.: 82-83°C were yielded.

### Example 85

### C-(3-{2-[4-(5-methoxy-pyrimidin-4-yl)-piperazin-1-yl]-ethyl}-1H-indol-5-yl)-N-methyl-methanesulfonamide

### Process A

### Methyl 3-{2-[4-(5-methoxy-pyrimidin-4-yl)-piperazin-1-yl]-ethyl}-5-methylsulfamoylmethyl-1H-indole-2-carboxylate

To 9.6 g (0.02 ml) of ethyl 5-methylsulfamoylmethyl-3-[2-(toluene-4-sulfonyloxy)-ethyl]-1H-indol-2-carboxylate (ES 2033577 A1) in 100 ml of acetonitrile were added 4.3 g (0.022 mol) of 5-methoxy-4-piperazin-1-yl-pyrimidine and 1.85 g (0.022 mol) of sodium bicarbonate. The reaction was held under reflux for 18 hours. It was concentrated to dryness. 100 ml of 2N NaOH were added and it was extracted with 3 x 100 ml of CH₂Cl₂. The organic phase was dried and concentrated. The residue was chromatographed over silica gel, eluting with CH₂Cl₂/EtOH 9/1 and yielding 6.7 g of a solid. IR(KBr): 1590; 1710 cm⁻¹
¹H-NMR: (200 MHz, CDCl₃): 9.12 (s, 1H); 8.33 (s, 1H); 7.90 (s, 1H); 7.70 (s, 1H); 7.36-7.33 (s.c., 2H); 4.60 (q, 1H); 4.34 (s, 2H); 3.95 (s, 3H); 3.87 (s, 3H); 3.85-3.77 (s.c., 4H) 3.33-3.20 (s.c., 2H); 2.74 (d, 3H); 2.73-2.60 (s.c., 6H)

### Process B

### 3-{2-[4-(5-methoxy-pyrimidin-4-yl)-piperazin-1-yl]-ethyl}-5-methylsulfamoylmethyl-1H-indole-2-carboxylic acid

To 5.5 g (0.011 mol) of the product prepared in Process A were added 2.18 g of 85% potassium hydroxide dissolved in 30 ml of distilled water. The reaction was held a 40°C for 2 hours. It was acidulated with acetic acid to pH=6. It was concentrated to dryness and the residue was treated with 10 ml of water and was filtered. 4.9 g (91.2%) of a beige solid were thereby yielded.
IR(KBr): 1590 cm⁻¹
¹H-NMR: (200MHz, DMSO): 11.4 (s.a., 1H); 8.26 (s, 1H); 8.04 (s, 1H); 7.62 (s, 1H); 7.37 (d, 1H); 7.20 (d, 2H);6.84 (s.a., 1H); 4.38 (s, 2H); 3.84 (s, 3H). 3.74-3.64 (s.c., 4H); 3.30-3.18 (s.a., 2H); 2.70-2.44 (s.c., 9H).

### Process C

### C-(3-{2-[4-(5-methoxy-pyrimidin-4-yl)-piperazin-1-yl]-ethyl}-1H-indol-5-yl)-N-methylmethanesulfonamide

To 3.9 g (8 mmol) of the product prepared in Process B in 20 ml of quinoline, were added 0.14 g of cuprous oxide. The mixture was heated a 180°C for 1 hour. It was allowed to cool and the catalyst was filtered off through decalite. The quinoline was removed by distillation in vacuo, and the residue was dissolved in 100 ml of CH₂Cl₂ and was washed with 50 ml of 2N NaOH, afterwards with 50 ml of water and finally with 50 ml of a saturated solution of sodium chloride. The organic phase was dried with sodium sulfate, was filtered and was concentrated. The residue was chromatographed over silica gel, eluting with CH₂Cl₂/EtOH 9/1, yielding 1.5 g (42.2%) of a solid.
IR(KBr): 1590 cm⁻¹
¹H-NMR:(200MHz, CDCl₃): 8.51 (s.a., 1H); 8.32 (s, 1H) 7.88 (s, 1H); 7.60 (s, 1H); 7.34 (d, 1H), 7.19 (d, 1H); 7.08 (s.a., 1H); 4.42 (q, 1H); 4.33 (s, 2H); 3.87 (s, 3H); 3.86-3.76 (s.c., 4H); 3.03-2.90 (s.c., 2H); 2.75-2.60 (s.c., 9H).

### Example 86

### C-(3-{3-[4-(4-chloro-5-phenyl-pyrimidin-4-yl)-piperazin-1-yl]-propyl}-1H-indol-5-yl)-N-methylmethanesulfonamide

Starting out from 1.38 g (5 mmol) of 4-chloro-5-phenyl-6-piperazin-1-yl-pyrimidine and 2.18 g (5 mmol) of 3-[5-[[(methylamino)sulfonyl]methyl]-1H-indol-3-yl]propyl-p-toluene-sulfonate and following the process described in Example 51 1.4 g (52%) of product in form of a white solid were yielded.
m.p: 76-77°C
IR(KBr): 1560; 1520; 1440 cm⁻¹.
¹H-NMR: 10.79 (s.a., 1H); 8.20 (s, 1H); 7.56-7.34 (m, 6H); 7.32 (d, 1H); 7.10 (s.a., 1H); 7.08 (d, d, 1H); 6,78 (q, 1H); 4,15 (s, 2H); 3,22-3,16 (m, 4H) 2.62 (t, 2H); 2.52 (d, 3H); 2.30-2.10 (m, 6H, 4H); 1.80-1.64 (m, 2H)

### Example 87

### C-(3-{3-[4-(5-phenyl-pyrimidin-4-yl)-piperazin-1-yl]-propyl}-1H-indol-5-yl)-N-methylmethanesulfonamide

Starting out from 1.2 g (5 mmol) of 5-phenyl-6-piperazin-1-yl-pyrimidine and 2.18 g (5 mmol) of 3-[5-[[(methylamino)sulfonyl]methyl]-1H-indol-3-yl]propyl p-toluenesulfonate and following the process described in Example 51, 1.6 g of product in form of white solid were yielded (63%).
m.p: 76-77°C
IR(KBr): 1590; 1540; 1450 cm⁻¹.
¹H-NMR: 10.8 (s.a., 1H); 8.6 (s, 1H); 8.18 (S, 1H); 7.52-7.35 (m, 6H); 7.32 (d, 1H) 7.13 (s.a, 1H); 7.08 (dd, 1H); 6.78 (q, 1H) 4.34 (S, 2H); 3.24-3.18 (m, 4H); 2.65 (t, 2H) 2.50 (d, 3H); 2.37 - 2.23 (m, 6H); 1.22-1.16 (m, 2H).

### 5HT1-like agonist activity of the synthesis products on the isolated rabbit saphenous vein preparation.

This study was conducted using the method described by Van Heuven-Nolsen (Van Heuven-Nolsen et al., Eur. J. Pharmacol. 191 (3); 375-382; 1990) and male New Zealand rabbits were used. The animals were sacrificed by exposure to excess CO₂. Thereafter the saphenous vein of each rear extremity was excised. 5 mm long segments were obtained from each vein. Each segment was placed, using appropriate supports, in an organ bath containing a Krebs-Henseleit solution aerated with carbogen and thermostatically controlled to 37°C. These conditions were held for 25 minutes, after which the preparation was subjected to a traction of 2 g at the same time as phenoxybenzamine (0.3 µM) and pargyline (500 µM) were added to the organ bath over 30 minutes. The phenoxybenzamine is an irreversible antagonist of the α-adrenoreceptors, whilst pargyline is a selective monoaminooxidase (MAO) inhibitor. At the end of these 30 minutes, successive washings were performed with Krebs solution every 7 minutes, for a further 30 minutes to remove the excess phenoxybenzamine. Thereafter, the preparation was subjected to an increasing contraction by the addition of cumulative doses of sumatriptan (10⁻⁹ at 3x10⁻⁵ M), followed by washing and allowing to rest for 30 minutes until the segment returns to its basal tension. At the end of this time, a further cumulative curve is plotted with the product to be studied. The pD2 (-log EC₅₀, the product concentration producing 50% of its maximum contraction) and the Eₘₐₓ (maximum contraction achieved) were calculated.

### Study of the binding to specific brain receptors (5HT_{1D}) by binding techniques.

This was performed as described by Weisberg & Teitler (Weisberg & Teitler, Drug Dev. res. 26; 225-234, (1992)) with slight modifications. A calf brain caudate nucleus homogenate was prepared in a Tris-HCl buffer solution. The homogenate was centrifuged and washed, the pellet was resuspended in the same buffer solution and centrifuged again. The pellet obtained was resuspended in Tris-HCl solution with 0.1% ascorbate and 10 µM pargyline. The tests were performed by incubating the suspension with ³H-5-HT, pindolol and mesulergide 1 µM. The test tubes were incubated and rapidly filtered, the radioactivity being measured. Sumatriptan was used as unmarked reference product.

| Ex. | PRODUCT | BINDING TESTS TO THE 5HT₁D RECEPTORS | RABBIT SAPHENOUS VEIN |
|---|---|---|---|
| | | | pD2 |
| 51 | VS-395 | 6.0xE-10 | 8.14±0.65 |
| 56 | VS-454 | 1.9xE-8 | 6.34±0.72 |
| 59 | VS-457 | 9.9xE-9 | 6.64±0.24 |
| 62 | VS-460 | 3.7xE-9 | 6.92±0.16 |
| 64 | VS-462 | 1.1xE-9 | 6.98±0.52 |
| 70 | VS-385 | 1.0xE-8 | 6.49±0.45 |
| 78 | VS-463 | 1.4xE-8 | 6.80±0.56 |
| 80 | VS-390 | 1.4xE-9 | 8.86±1.05 |
| 83 | VS-365 | 1.5xE-9 | 6.14±0.98 |
| 85 | VS-382 | 7.0xE-6 | 5.58±0.21 |
| 87 | VS-472 | 1.3xE-9 | 6.55±0.32 |
| | SUMATRIPTAN | 2.2xE-8 | 6.46±0.17 |
| | SEROTONIN | 2.3xE-9 | 7.31±0.08 |

## Claims

1. Indole derivatives useful for the treatment of migraine, of formula I 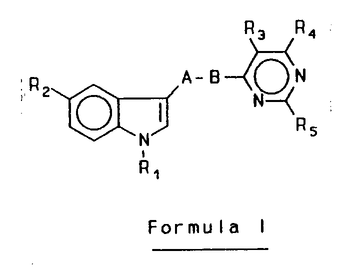 where
R₁ is an atom of hydrogen or a C₁₋₆ lower alkyl or C₁₋₆ lower acyl group;
R₂ is an atom of hydrogen or halogen or a C₁₋₆ lower alkyl group, hydroxy, C₁₋₆ lower alkoxy; cyano, carboxamido, carboxy, alkoxycarbonyl, phenyl, optionally substituted phenyloxy or a -(CH₂)ₙ-R₆ group where n is 1, 2 or 3 and R₆ is cyano, nitro, optionally substituted phenyl, carboxy, -CO₂R₇; -CONR₇R₈; -SO₂NR₇R₈; -COR₇; -SO₂R₇, where R₇ and R₈ are independently an atom of hydrogen or a C₁₋₆ lower alkyl group, or R₈ and R₈ together with the N may form a ring having 4 to 7 members with one or more hetero atoms, said ring being optionally substituted by an alkyl group, hydroxy, alkoxy, alkoxycarbonyl, cyano, nitro, carboxamido, sulfonamido, phenyloxy, benzyloxy;
R₃, R₄ and R₅
are independent of each other and are an atom of hydrogen or halogen or a C₁₋₆ lower alkyl group, hydroxy, C₁₋₆ lower alkoxy; optionally substituted phenyl; optionally substituted phenyloxy; benzyloxy optionally substituted in the phenyl ring; NR₁₁R₁₂, where R₁₁ and R₁₂ are independent of each other and are an atom of hydrogen or a C₁₋₆ lower alkyl group, cyano, nitro, carboxy, alkoxycarbonyl, carboxamido or a -(CH₂)ₙ-R₆ group, where n and R₆ are as hereinbefore defined;
R₃ nevertheless cannot be hydrogen or lower alkoxy when R₄ and R₅ are hydrogen and B is a piperazine ring;
A is either -(CH₂)ₘ- alkylidene group optionally substituted with hydroxy, m being an integer ranging from 1 to 5, or a C₁₋₅ alkenyl group;
B is a piperazine ring 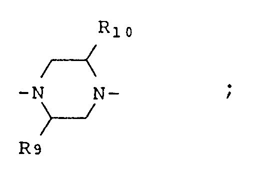 an aminopyrrolidine ring 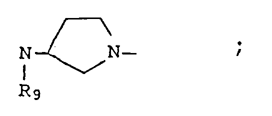 a pyrrolidineamino ring 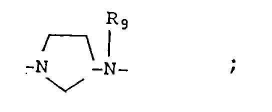 a piperidine ring 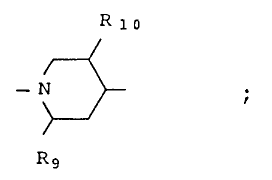 or ethylenediamino -NR₉-CH₂-CH₂-NR₁₀-
R₉ and R₁₀
are independent of each other and are an atom of hydrogen or a C₁₋₆ lower alkyl group, or a pharmaceutically acceptable salt and/or a solvate and/or a stereoisomer thereof.

2. Indole derivatives as defined in claim 1, wherein R₂ is a -CH₂-SO₂NR₇R₈ group.

3. Indole derivatives as defined in claim 1, wherein R₃ and R₄ are -OCH₃ groups.

4. Indole derivatives as defined in claim 1, having the chemical name:
C-(3-{3-[4-(5,6-dimethoxy-pyrimidin-4-yl)-piperazin-1-yl]-propyl}-1H-indol-5-yl)-N-methylmethanesulfonamide
C-(3-{3-[1-(5-methoxy-pyrimidin-4-yl)-pyrrolidin-3-yl-amino]-propyl}-1H-indol-5-yl)-N-methylmethanesulfonamide
C-(3-{3-[1-(5,6-dimethoxy-pyrimidin-4-yl)-pyrrolidin-3-yl-amino]-propyl}-1H-indol-5-yl)-N-methylmethanesulfonamide
C-(3-{3-[3-(5-methoxy-pyrimidin-4-yl-amino)-pyrrolidin-1-yl]-propyl}-1H-indol-5-yl)-N-methylmethanesulfonamide
C-(3-{3-[3-(5,6-dimethoxy-pyrimidin-4-yl-amino)-pyrrolidin-1-yl]-propyl}-1H-indol-5-yl)-N-methylmethanesulfonamide
C-[3-(3-{2-[(5-methoxy-pyrimidin-4-yl)-methyl-amino]-ethylamino}-propyl)-1H-indol-5-yl]-N-methylmethanesulfonamide
C-[3-(3-{2-[(5,6-dimethoxy-pyrimidin-4-yl)-methyl-amino]-ethylamino}-propyl)-1H-indol-5-yl]-N-methylmethanesulfanamide
C-{3-[3-({2-[(5-methoxy-pyrimidin-4-yl)-methylamino]-ethyl}-methylamino)-propyl]-1H-indol-5-yl}-N-methylmethanesulfonamide
C-{3-[3-({2-[(5,6-dimethoxy-pyrimidin-4-yl)-methylamino]-ethyl}-methylamino)-propyl]-1H-indol-5-yl}-N-methylmethanesulfonamide
C-[3-(3-{2-[(5,6-dimethoxy-pyrimidin-4-yl)-amino]-ethylamino}-propyl)-1H-indol-5-yl}-N-methylmethanesulfonamide
C-(3-{3-[4-(6-methylamino-5-methoxy-pyrimidin-4-yl)-piperazin-1-yl]-propyl}-1H-indol-5-yl)-N-methylmethanesulfonamide
3-{3-[4-(5,6-dimethoxy-pyrimidin-4-yl)-piperazin-1-yl]-propyl}-5-fluoro-1H-indole
3-{3-[4-(5,6-dimethoxy-pyrimidin-4-yl)-piperazin-1-yl]-propyl}-5-methoxy-1H-indole
C-(3-{3-[3-(5,6-dimethoxy-pyrimidin-4-yl)-piperazin-1-yl]-1-methyl}-1H-indol-5-yl)-N-methylmethanesulfonamide
C-(3-{3-[3-(5,6-dimethoxy-pyrimidin-4-yl)-piperazin-1-yl]-1-methyl}-1H-indol-5-yl)-N,N-dimethylmethanesulfonamide
C-(3-{3-[4-(5-phenyl-pyrimidin-4-yl)-piperazin-1-yl]-propyl}-1H-indol-5-yl)-N-methylmethanesulfonamide

5. A composition comprising: (a) a pharmaceutically effective amount of an indole derivative as defined in any one of claims 1 to 4; and (b) a pharmaceutically acceptable excipient.

6. The use of an indole derivative as defined in any one of claims 1 to 4 for the preparation of a therapeutically applicable antimigraine drug.

7. A pharmaceutical formulation as defined in any one of claims 5 or 6, for use as an antimigraine formulation.
